**Europäisches Patentamt**

**European Patent Office**

**Office européen des brevets**

(19)

(11) **EP 1 628 229 A1**

(12) **EUROPEAN PATENT APPLICATION**
published in accordance with Art. 158(3) EPC

(43) Date of publication:
22.02.2006 Bulletin 2006/08

(51) Int Cl.:
*G06F 17/30* (1995.01)   *G06T 11/20* (1995.01)

(21) Application number: 03733127.9

(22) Date of filing: 28.05.2003

(86) International application number:
PCT/JP2003/006676

(87) International publication number:
WO 2004/107210 (09.12.2004 Gazette 2004/50)

(84) Designated Contracting States:
AT BE BG CH CY CZ DE DK EE ES FI FR GB GR HU IE IT LI LU MC NL PT RO SE SI SK TR

(71) Applicant: Celestar Lexico-Sciences, Inc.
Chiba-shi,
Chiba 261-8501 (JP)

(72) Inventors:
• NITTA, Kiyoshi
c/o Celestar Lexico-Sciences, Inc.
Chiba-shi,
Chiba 261-8501 (JP)

• SUZUKI, Ichiro
c/o Celestar Lexico-Sciences, Inc.
Chiba-shi,
Chiba 261-8501 (JP)

(74) Representative: HOFFMANN EITLE
Patent- und Rechtsanwälte
Arabellastrasse 4
81925 München (DE)

(54) **PATHWAY INFORMATION DISPLAY DEVICE**

(57)    A pathway information display apparatus (100) and an external system (200) that provides external databases related to pathway information and the like including genetic control pathways, metabolic pathways, information transduction pathways, and the like, external programs for a homology search and the like, are communicably connected to each other through a network (300).

FIG.2

## Description

TECHNICAL FIELD

The present invention relates to a pathway information display apparatus, a pathway information display method, a program, and a recording medium. More specifically, the invention relates to a pathway information display apparatus, a pathway information display method, a program, and a recording medium for automatically drawing a binary relation model.

The present invention also relates to a directed graph layout apparatus, a directed graph layout method, a program, and a recording medium. More specifically, the invention relates to a directed graph layout apparatus, a directed graph layout method, a program, and a recording medium capable of expressing flows of edges of a directed graph.

BACKGROUND ART

(I) Techniques for creating a model of a metabolic circuit, a signal-transfer pathway, or a genetic control pathway in the form of a graph have been conventionally proposed in the bioinformatics field.

For example, P.Karp and S.M.Paley, "Automated drawing of metabolic pathways" (in Third International Conference on Bioinformatics and Genome Research (H.Lim, C.Cantor, and R.Robbins, eds. 1994) discloses a technique for creating models of metabolic pathways in the form of graphs and drawing the graphs.

Tomihiro Koike and Andrey Rzhetsky, "A graphic editor for analyzing signal-transfer pathways" (GENE 259, pp.235-244, 2000) discloses a technique for creating models of signal-transfer pathways in the form of graphs and drawing and editing the graphs.

Moritz Y.Becker and Isabel Rojas. "A graph layout algorithm for drawing metabolic pathways" (Bioinformatics, Vol.17, No5, pp.461-467, 2001) discloses a technique for creating models of metabolic pathways in the form of graphs and drawing the graphs.

The conventional techniques for creating models of metabolic pathways and the like in the form of graphs and drawing the graphs have, however, the following problems. These conventional techniques can automatically draw a relatively simple and small-sized pathway graph. However, they cannot automatically draw a complicated pathway graph and a medium or large-sized pathway graph for reasons including one that it takes a long time to perform calculations.

Furthermore, in each of the pathway graphs automatically drawn by the conventional techniques, nodes, edges, and label description regions thereof are often overlapped, which makes it disadvantageously difficult to view the graph.

Various tools have been developed for user's manually drawing pathway information in the form of graphs. In drawing graphs of complicated pathways or medium or large-sized pathways, it is necessary to uniformly draw them while appropriately clustering internode relations (that is, to draw more relevant nodes to be closer to each other) and avoid overlapping of nodes. They require a skilled technique for handling the tools. Therefore, even if one of these tools is used, it takes a lot of time and labor, with the result that it is disadvantageously difficult to create such graphs.

As described above, conventional pathway graph drawing systems and the like in the bioinformatics field are confronted with many problems. The conventional techniques are, therefore, inferior in friendliness to both users and drawers and in utilization efficiency.

The conventional techniques and the problems to be solved by the present invention explained so far are not limited to bioinformatics systems for drawing graphs of pathway information such as genetic control pathways, metabolic pathways, and signal-transfer pathways. The same is true for all systems for drawing graphs of pathway information in every field.

It is, therefore, an object of the present invention to provide a pathway information display apparatus, a pathway information display method, a program, and a recording medium capable of automatically drawing a graph of a complicated pathway or a medium-sized or large-sized pathway.

(II) Meanwhile, in the field of information processing, there have been known techniques for automatically drawing a graph based on a spring layout system using force-directed scheme or a magnetic spring system (Japanese Patent Application Laid-open No. H8-30799) capable of making directions of edges of a directed graph unidirectional.

However, the conventional techniques have the following problems. If a model of a pathway such as a metabolic pathway is to be created in the form of a directed graph (a graph having directed edges), various graph layouts are present such as a graph layout capable of expressing a graph of, for example, a signal-transfer circuit in one direction and a graph layout capable of expressing a graph of, for example, a metabolic circuit in a circular direction mainly due to presence of a forward path. Due to this, with the technique for making a direction of a graph layout uniform, e.g., the conventional magnetic spring system, a graph having locally different directions cannot be laid out.

Namely, if a directed graph in which a pathway on which a forward path is present and a pathway on which a cyclic path is not present is to be laid out using the conventional techniques, it is necessary to do so by a combination of various methods or by manually making fine adjustments. With the conventional techniques, therefore, it is impossible to efficiently lay out a directed graph by a simple method.

Various tools have been developed for user's manually drawing pathway information in the form of graphs. In drawing graphs for complicated pathways or medium or large-sized pathways, it is necessary to uniformly draw them while appropriately clustering internode relations (that is, to draw more relevant nodes to be closer to each other) and avoid overlapping of nodes. They require a skilled technique for handling the tools. Therefore, even if one of these tools is used, it takes a lot of time and labor, with the result that it is disadvantageously difficult to create such graphs.

As described above, conventional pathway graph drawing systems and the like in the bioinformatics field are confronted with many problems. The conventional techniques are, therefore, inferior in friendliness to both users and drawers and in utilization efficiency.

The conventional techniques and the problems to be solved by the present invention explained so far are not limited to bioinformatics systems for drawing graphs of pathway information such as genetic control pathways, metabolic pathways, and signal-transfer pathways. The same is true for all systems for drawing graphs for pathway information in every field.

It is, therefore, an object of the present invention to provide a directed graph layout apparatus, a directed graph layout method, a program, and a recording medium capable of efficiently laying out a directed graph by a simple method if a pathway on which a forward path is present and a pathway on which a cyclic path is not present are mixed in the directed graph.

DISCLOSURE OF INVENTION

(I) To achieve such an object, a pathway information display apparatus, a pathway information display method, and a program according to the present invention include a binary relation model creation unit (a binary relation model creation step) of creating a binary relation model of pathway information configured by nodes and edges; a repulsive force calculation unit (a repulsive force calculation step) of calculating a repulsive force generated between the nodes within a certain distance; an attractive force calculation unit (an attractive force calculation step) of calculating an attractive force generated between the nodes connected to each other by the edges; a repulsion field calculation unit (a repulsion field calculation step) of calculating the repulsive force and the attractive force by setting a repulsion field for a preset peripheral region of the nodes; and a graph drawing unit (a graph drawing step) of determining a coordinate position of each of the nodes based on the repulsive force calculated by the repulsive force calculation unit (the repulsive force calculation step), the attractive force calculated by the attractive force calculation unit (the attractive force calculation step), and the repulsive force and the attractive force in the repulsion field calculated by the repulsion field calculation unit (the repulsion field calculation step), using a spring layout system, and drawing the nodes and the edges in the form of a graph, wherein the pathway information is displayed as the graph using the binary relation model.

According to the apparatus, the method, and the program, a binary relation model of pathway information configured by nodes and edges is created. The pathway information includes information on genetic control pathways, metabolic pathways, signal-transfer pathways or the like. By mapping main information on a binary relation, the complicated pathway information can be simplified into a model and integrally expressed. Furthermore, a repulsive force generated between the nodes within a certain distance is calculated, and an attractive force generated between the nodes connected to each other by the edges is calculated. A coordinate position of each node is determined based on the repulsive force and the attractive force, and the nodes and the edges are drawn in the form of a graph. By doing so, even if the pathway information is medium or large-sized pathway information or complicated pathway information, a graph that is readable to some extent can be automatically drawn within a certain time.

If the layout is executed only by forces based on the repulsive force and the attractive force between each node pair, the nodes are often so close to each other to thereby make the edges and their labels difficult to distinguish from one another. According to the present invention, therefore, the repulsion field is set for a preset peripheral region of the nodes so as to prevent every node pair from being too close to each other. That is, according to the invention, the repulsion field is set for the preset peripheral region of the nodes and the repulsive force and the attractive force are then calculated. Based on the calculated repulsive force and attractive force, and the repulsive force and attractive force in the repulsion field, the coordinate position of each node is determined by the spring layout system, the nodes and the edges are drawn in the form of a graph, and the pathway information is displayed as a graph using the binary relation model. Therefore, overlapping of the nodes and the edges themselves and their labels is avoided, thereby further improving readability of the graph.

According to the pathway information display apparatus, the pathway information display method, and the program according to the next invention, which are based on the pathway information display apparatus, the pathway information display method, and the program explained above, the repulsive force calculation unit calculates the repulsive force and the attractive force in the repulsion field (at the repulsive force calculation step, the repulsive force and the attractive force in the repulsion field are calculated) by performing both or one of weakening of the attractive force for the peripheral regions of the nodes and strengthening the repulsive force between the nodes located in the peripheral regions of the nodes.

This shows one example of the calculation of the repulsion field more specifically. According to the apparatus, the method, and the program, the repulsion field is calculated by performing both of or one of weakening of the attractive force for the peripheral regions of the nodes (including not generating the attractive force) and/or strengthening of the repulsive force between every node pair located within the node peripheral region. Based on the forces, coordinate positions of the entire nodes are determined so that an entire force (e.g., an energy value that is the sum of absolute values of synthetic vectors for all edges) can realize the system in a minimum energy state by the spring layout system. The nodes and the edges are drawn in the form of a graph.

The pathway information display apparatus, the pathway information display method, and the program according to the next invention, which are based on the pathway information display apparatus, the pathway information display method, and the program explained above, further include a boundary determination unit (a boundary determination step) of determining whether the coordinate position of the each node falls within a preset drawing region, and of, if the coordinate position is out of the drawing region, moving the coordinate position of the node to a boundary of the drawing region.

According to the apparatus, the method, and the program, it is determined whether a coordinate position of each node falls within a preset drawing region. If the node coordinate position is out of the drawing region, the node coordinate position is moved to a boundary of the drawing region. Therefore, the pathway information can be always drawn within the drawing region.

The pathway information display apparatus, the pathway information display method, and the program according to the next invention, which are based on the pathway information display apparatus, the pathway information display method, and the program explained above, further include a dynamic parameter control unit (a dynamic parameter control step) of controlling the repulsion field generated between the nodes to be narrowed and the attractive force generated between the nodes to be strengthened before a preset time passes, controlling the repulsion field of the nodes to be widened, the attractive force generated between the nodes to be weakened, and the repulsive force generated between the nodes to be strengthened, and thereby controlling the graph to be drawn so that relevant nodes are closer to each other before the preset time passes and so that the closer nodes are dispersed from each other after passage of the preset time.

According to the apparatus, the method, and the program, the node repulsion field is controlled to be narrowed and the attractive force generated between the nodes is controlled to be strengthened before a preset time passes, and the node repulsion field is controlled to be widened. In addition, the attractive force generated between the nodes is controlled to be weakened, and the repulsive force generated between the nodes is controlled to be strengthened after the preset time passes. It is thereby possible to control the graph to be drawn so that the relevant nodes are closer to each other before the time passes and so that the closer nodes are dispersed after the time passes.

The pathway information display apparatus, the pathway information display method, and the program according to the next invention, which are based on the pathway information display apparatus, the pathway information display method, and the program explained above, further include an energy calculation unit (an energy calculation step) of calculating an energy by calculating a sum of absolute values of synthetic vectors based on the nodes and the edges drawn by the graph drawing unit (at the graph drawing step), wherein the dynamic parameter control unit repeatedly performs the operation (the dynamic parameter control step is repeatedly executed) until the energy calculated by the energy calculation unit (at the energy calculation step) is lower than a preset value, or until the energy becomes minimum.

According to the apparatus, the method, and the program, the energy is calculated by calculating a sum of absolute values of synthetic vectors based on the drawn nodes and edges. The dynamic parameter control is repeatedly executed until the calculated energy is lower than a preset value, or until the energy becomes minimum. It is thereby possible to calculate the layout so as to realize the system in a low energy state to a certain extent or in a minimum energy state.

The pathway information display apparatus, the pathway information display method, and the program according to the next invention, which are based on the pathway information display apparatus, the pathway information display method, and the program explained above, further include a dummy node setting unit (a dummy node setting step) of, if a connection destination of one of the edges for one of the nodes is another one of the edges, setting a dummy node on the another edge, wherein the graph drawing unit draws the graph (at the graph drawing step, the graph is drawn) while using a portion of the dummy node as a bent point.

According to the apparatus, the method, and the program, if a connection destination of an edge for one of the nodes is another one of the edges, a dummy node on another edge is set, and the graph is drawn while using a portion of the dummy node as a bent point. It is, therefore, possible to provide the pathway information display apparatus, the pathway information display method, the program, and the recording medium capable of improving the readability of the graph if the edge is connected to another edge.

Furthermore, the present invention relates to a recording medium, and the recording medium according to the invention records the program explained above.

According to the recording medium, by causing a computer to read the program recorded in the recording medium and execute the program, the program can be realized using the computer. The same advantages as those of the respective methods can be obtained.

(II) To achieve the object, a directed graph layout apparatus, a directed graph layout method, and a program are a directed graph layout apparatus, a directed graph layout method, and a program for automatically laying out a directed graph configured by nodes and directed edges, including a synthetic vector generation unit (a synthetic vector generation step) of generating a synthetic vector of all unit vectors, the unit vectors being the directed edges connected to each of the nodes; an angle correcting force generation unit (an angle correcting force generation step) of generating an angle correcting force for the each node serving as a connection destination node of the directed edges in a direction in which an angle between the synthetic vector generated by the synthetic vector generation unit (at the synthetic vector generation step) and each of the unit vectors is smaller and in a direction perpendicular to the each unit vector; an energy calculation unit (an energy calculation step) of calculating an energy by calculating a sum of the angle correcting forces for all the nodes; and an optimization control unit (an optimization control step) of changing a position of each of the nodes so as to optimize the energy calculated by the energy calculation unit (at the energy calculation step).

According to the apparatus, the method, and the program, a synthetic vector of all unit vectors is generated, with the directed edges connected to each of the nodes assumed as the unit vectors. An angle correcting force is generated for the each node serving as a connection destination node of the directed edges in a direction in which an angle between the generated synthetic vector and each of the unit vectors is smaller and in a direction perpendicular to the each unit vector. Energy is calculated by calculating a sum of the angle correcting forces for all the nodes, and a position of each of the nodes is changed so as to optimize the calculated energy. It is, therefore, possible to automatically draw a graph in which flows of edges of a directed graph are expressed.

Namely, a directed graph in which a pathway on which a forward path is present and a pathway on which a cyclic path is not present are mixed can be automatically laid out so that the forward path part is closer to a circle and directions of edges coincide with one another on a part other than the forward path. The directed graph layout can be realized efficiently by a simple method.

Furthermore, according to the present invention, the drawing region can be generally effectively used, as compared with an instance of drawing a graph using the magnetic spring system.

The directed graph layout apparatus, the directed graph layout method, and the program according to the next invention, which are based on the directed graph layout apparatus, the directed graph layout method, and the program explained above, further include a unit vector multiplication unit (a unit vector multiplication step) of multiplying the each unit vector of each of the directed edges by a coefficient according to a type of the each directed edge, wherein the synthetic vector generation unit generates the synthetic vector (at the synthetic vector generation step, the synthetic vector is generated) using the each unit vector multiplied by the coefficient by the unit vector multiplication unit (at the unit vector multiplication step).

This shows one example of synthesizing the unit vectors more specifically. According to the apparatus, the method, and the program, each unit vector of each of the directed edges is multiplied by a coefficient according to a type of the directed edge, and the synthetic vector is generated using the unit vectors each multiplied by the coefficient. Therefore, a weight can be given to the directivity of each directed edge according to the type of the directed edge. For instance, as types of the edges, a normal route (a route in a stationary state), an abnormal route (a route appearing under abnormal conditions), and the like are set, and heavier weight is given to the normal route. It is thereby possible to lay out the graph so that the directivity of each edge is along the normal route.

According to the directed graph layout apparatus, the directed graph layout method, and the program according to the next invention, which are based on the directed graph layout apparatus, the directed graph layout method, and the program explained above, the angle correcting force is a preset constant.

This shows one example of the angle correcting force more specifically. According to the apparatus, the method, and the program, the angle correcting force is a preset constant. The angle correcting force can be obtained without making complicated calculations, and without need of lots of calculation time and calculation amount.

According to the directed graph layout apparatus, the directed graph layout method, and the program according to the next invention, which are based on the directed graph layout apparatus, the directed graph layout method, and the program explained above, the angle correcting force is changed according to the angle.

This shows one example of the angle correcting force more specifically. According to the apparatus, the method, and the program, the angle correcting force is changed according to the angle. The optimization can be, therefore, efficiently executed in the initial state.

The directed graph layout apparatus, the directed graph layout method, and the program according to the next invention, which are based on the directed graph layout apparatus, the directed graph layout method, and the program explained above, further includes an attractive force calculation unit (an attractive force calculation step) of calculating an attractive force generated between the nodes connected to each other by the edges; a repulsive force calculation unit (a repulsive force calculation step) of calculating a repulsive force generated between the nodes within a certain distance, wherein the energy calculation unit calculates the energy (at the energy calculation step, the energy is calculated) by calculating a sum of the attractive forces, the repulsive forces, and the angle correcting forces for all the nodes.

According to the apparatus, the method, and the program, an attractive force generated between the nodes connected to each other by the edges is calculated, and a repulsive force generated between the nodes within a certain distance is calculated. The energy is calculated by calculating a sum of the attractive forces, the repulsive forces, and the angle correcting forces for all the nodes. When the angle adjustment is made, all the directed edges are to be overlaid on a certain line (synthetic vector). However, by utilizing a force-directed layout scheme such as the spring layout system together with the present invention, the repulsive force generated between nodes closer to each other to some extent is stronger, and the nodes repel each other. It is, therefore, possible to prevent different edges from being completely overlaid.

Furthermore, the present invention relates to a recording medium, and the recording medium according to the invention records the program explained above.

According to the recording medium, by causing a computer to read the program recorded in the recording medium and execute the program, the program can be realized using the computer. The same advantages as those of the respective methods can be obtained.

BRIEF DESCRIPTION OF DRAWINGS

Fig. 1 is a conceptual view of an outline of the basic principle of the present invention;
Fig. 2 is a block diagram of one example of a system to which the present invention is applied;
Fig. 3 is a conceptual view of a binary relation model;
Fig. 4 is one example of information stored in a binary relation model database 106a;
Fig. 5 is a flowchart of one example of main processing performed by the system according to the embodiment;
Fig. 6 is a flowchart of one example of a spring layout processing performed by the system according to the embodiment;
Fig. 7 is a flowchart of one example of a dynamic parameter control processing according to the embodiment of the present invention;
Fig. 8 is a flowchart of one example of a dummy node processing performed by the system according to the embodiment;
Fig. 9 is one example of a binary relation model creation screen displayed on an output device 114 of a pathway information display apparatus 100;
Fig. 10 is a principle block diagram of the basic principle of the present invention;
Fig. 11 depicts a concept of an angle adjustment using an angle correcting force according to the present invention;
Fig. 12 is one example of a directed graph of a metabolic pathway or the like on which a forward path is present;
Fig. 13 is one example of a directed graph of a signal-transfer pathway or the like on which a cyclic path is not present;
Fig. 14 is a block diagram of one example of a configuration of a system to which the present invention is applied;
Fig. 15 is a conceptual view of a binary relation model;
Fig. 16 is one example of information stored in a binary relation model database 1106a;
Fig. 17 is a flowchart of one example of the main processing performed by the system according to the embodiment;
Fig. 18 is a flowchart of one example of a directed graph layout processing performed by the system according to the embodiment;
Fig. 19 is a flowchart of one example of an angle correcting force calculation processing performed by an angle adjustment unit 1102d;
Fig. 20 is one example of a directed graph output screen displayed on an output device 1114 of a directed graph layout apparatus 1100; and
Fig. 21 is one example of a binary relation model creation screen displayed on the output device 1114 of the directed graph layout apparatus 1100.

BEST MODE(S) FOR CARRYING OUT THE INVENTION

(I) Embodiments of a pathway information display apparatus, a pathway information display method, a program, and a recording medium according to the present invention will be explained below in detail with reference to the accompanying drawings. Note that the invention is not limited by the embodiments.

In the following embodiments, an instance that the present invention is applied to a bioinformatics system that draws a graph of pathway information such as a genetic control pathway, a metabolic pathway or a signal-transfer pathway will be explained. However, the invention is not limited to the instance, and can be similarly applied to a system that draws a graph of pathway information in every field.

[OUTLINE OF THE PRESENT INVENTION]

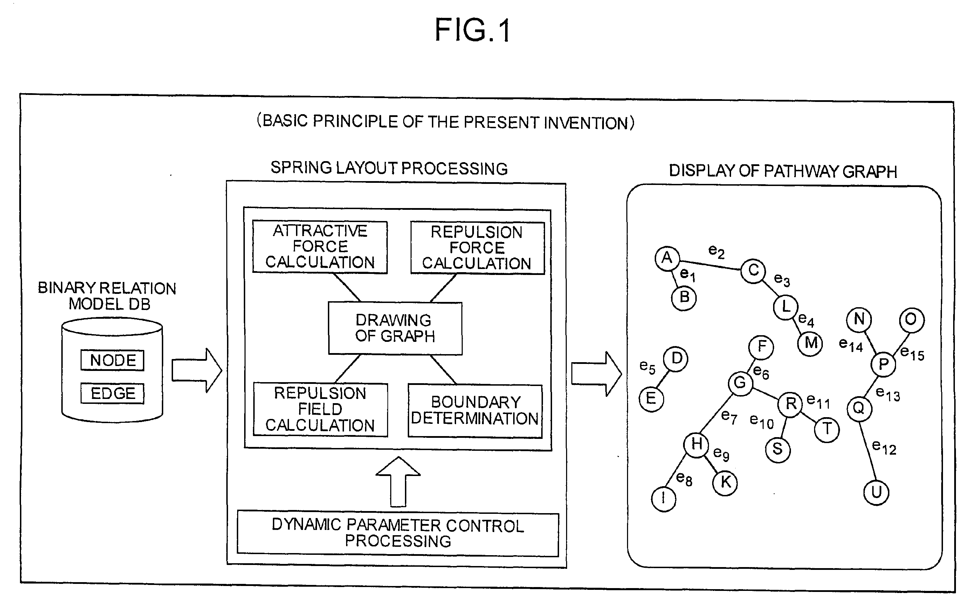

The outline of the basic principle of the present invention will be explained first, and a configuration, processing and the like of the invention will be explained next in detail. Fig. 1 is a conceptual view of the outline of the basic principle of the present invention.

According to the present invention, a binary relation model of pathway information configured by nodes and edges is created first to thereby construct a binary relation model database. The "nodes" express matters or phenomena. The "edges" express various internode relations. The pathway includes a node serving as a start point and a node or an edge serving as an end point, and expresses a binary relation from the start point to the terminal point. For instance, a node expresses either a matter such as a gene, a protein, an amino acid, an enzyme, a coenzyme or the like, or a phenomenon such as an enzyme reaction or a metabolic reaction. An edge expresses a relation between the start point and the end point such as formation, inhibition or bonding. Normally, nodes and edges are expressed in different forms according to types of pathways.

A spring layout system is then executed to nodes (A to U in Fig. 1) and edges ($e_1$ to $e_{15}$ in Fig. 1) defined in a binary relation model database to thereby draw a graph.
The "spring layout" is a layout called "force-directed layout" among ordinary undirected graph drawing methods. Nodes are replaced by rings and edges are replaced by springs, and a layout is calculated so that forces of the springs (a repulsive force and an attractive force) acting on the rings can realize a minimum energy state of the system. Namely, according to the present invention, a repulsive force and an attractive force between nodes are calculated according to a distance between the nodes. Node coordinate positions are determined based on the calculated forces, and the nodes and the edges are drawn in the form of a graph. The "repulsive force" is a force generated between every pair of nodes present within a certain distance, and the nodes are dispersed and overlaps of nodes are removed by the repulsive force. The "attractive force" is a force generated between every pair of nodes present within the certain distance. The nodes connected to each other by an edge by the attractive force are closer to each other. Therefore, relevant nodes can be made closer and easily discriminated from irrelevant nodes.

If the spring layout is executed only by forces based on the repulsive force and the attractive force between each node pair, the nodes are often so close to each other to thereby make the edges and their labels difficult to distinguish from each other. According to the present invention, therefore, a "repulsion field" is set for a preset peripheral region of the nodes so as to prevent every node pair from being too close to each other. That is, according to the present invention, the repulsive force is calculated by performing both of or one of weakening of the attractive force for the node peripheral region (including not generating the attractive force) and strengthening of the repulsive force between every node pair located within the node peripheral region. Based on the forces, coordinate positions of the entire nodes are determined so that an entire force (e.g., an energy value that is a sum of absolute values of synthetic vectors for all edges) can realize the minimum energy of the system in the spring layout. Thereafter, the nodes and the edges are drawn in the form of a graph.

Furthermore, according to the present invention, it is determined whether a coordinate position of each node falls within a preset drawing region. If the node coordinate position is out of the drawing region, the node coordinate position is moved to a boundary of the drawing region. The pathway information can be thereby always drawn within the drawing region. By thus setting the drawing region in advance, a limit can be set to a repulsive force acting distance. Accordingly, a phenomenon that nodes are permanently and continuously apart from each other as seen in the ordinary spring layout can be avoided. If a node movement is inhibited on a window boundary, a phenomenon that many nodes concentrate on the boundary can be avoided.

Furthermore, according to the present invention, the node repulsion field is controlled to be narrowed and the attractive force between the nodes is controlled to be strengthened before a preset time passes. After the preset time passes, the node repulsion field is controlled to be widened, the attractive force between the nodes is controlled to be weakened, and the repulsive force between the nodes is controlled to be strengthened. It is thereby possible to dynamically perform a parameter control and draw a graph while making relevant nodes closer to each other before the preset time passes and making the closer nodes dispersed after the preset time passes (a dynamic parameter control processing). The energy is calculated by calculating the sum of absolute values of synthetic vectors based on the nodes and the edges. The energy calculation is repeated until the energy is lower than a preset value, or the energy becomes minimum. By executing the dynamic parameter control processing, the relevant nodes can be made closer in a short time and appropriate clustering can be performed even for large-scale pathway information or complicated pathway information before the preset time passes. In addition, most of edge crossings are removed, so that chances for causing the energy to fall into a local minimum layout can be reduced. Besides, after the passage of the preset time, readability can be improved even for the complicated pathway information.

[SYSTEM CONFIGURATION]

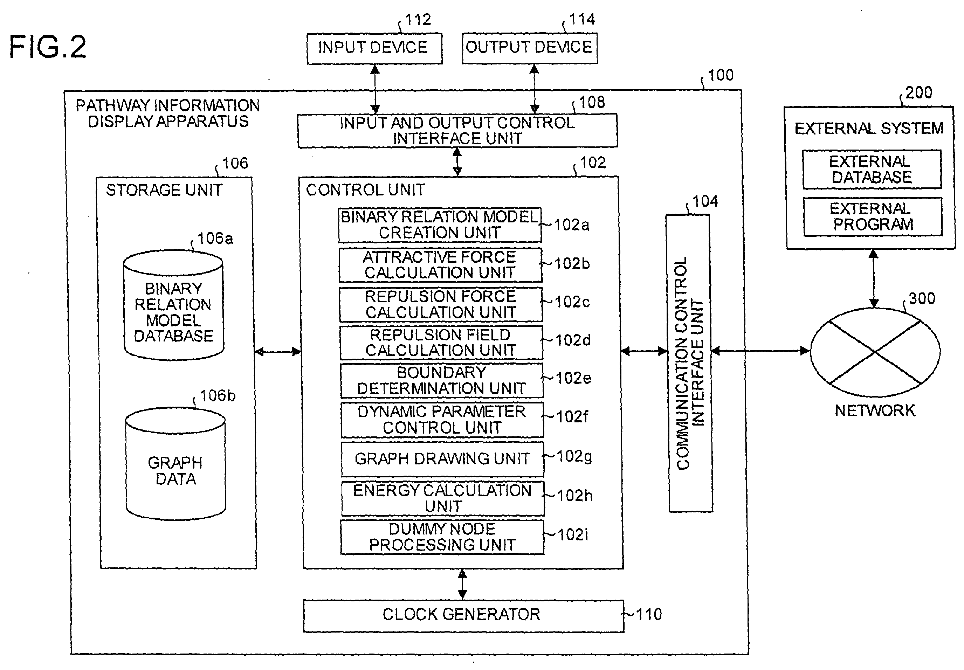

A configuration of a system to which the present invention is applied will be explained. Fig. 2 is a block diagram of one example of the configuration of the system to which the present invention is applied. In Fig. 2, only elements related to the invention are conceptually shown among all constituent elements of the system. The system is roughly configured such that a pathway information display apparatus 100 and an external system 200 are communicably connected to each other through a network 300. The external system 200 provides pathway information and the like including external databases related to genetic control pathways, metabolic pathways, and information transduction pathways, external programs and the like for a homology search, and the like.

In Fig. 2, the network 300, which functions to connect the pathway information display apparatus 100 to the external system 200, is, for example, the Internet.

In Fig. 2, the external system 200 is connected to the pathway information display apparatus 100 through the network 300. The external system 200 has a function to provide users with websites for executing the external databases related to the pathway information and the like and the external programs for the homology search, a motif search, and the like.

The external system 200 can be configured as a WEB server, an ASP server or the like, and a hardware configuration of the external system 200 can be configured by an information processing apparatus such as a commercially available workstation or personal computer as well as their additional apparatuses. Functions of the external system 200 are realized by a CPU, a disk device, a memory device, an input device, an output device, a communication control apparatus, and the like as well as programs for controlling these apparatuses and the like.

In Fig. 2, the pathway information display apparatus 100 generally includes a control unit 102 such as a CPU, a communication control interface unit 104, an input and output control interface unit 108, a clock generator 110, and a storage unit 106. The communication control interface unit 104 integrally controls entirety of the pathway information display apparatus 100. The communication control interface unit 104 is connected to a communication device (not shown), e.g., a router connected to a communication line or the like. The input and output control interface unit 108 is connected to the input device 112 and the output device 114. The clock generator 110 generates a system clock. The storage unit 106 stores various databases and tables (a binary relation model database 106a and a graph data 106b). These constituent elements are communicably connected to the network 300. Furthermore, the pathway information display apparatus 100 is communicably connected to the network 300 through the communication device such as a router and a wired or wireless communication line such as a dedicated line.

The various databases and tables (the binary relation model database 106a and the graph data 106b) stored in the storage unit 106 are storage means such as a fixed disk device and the like, and store various programs, tables, databases, webpage files, and the like used for various processing.

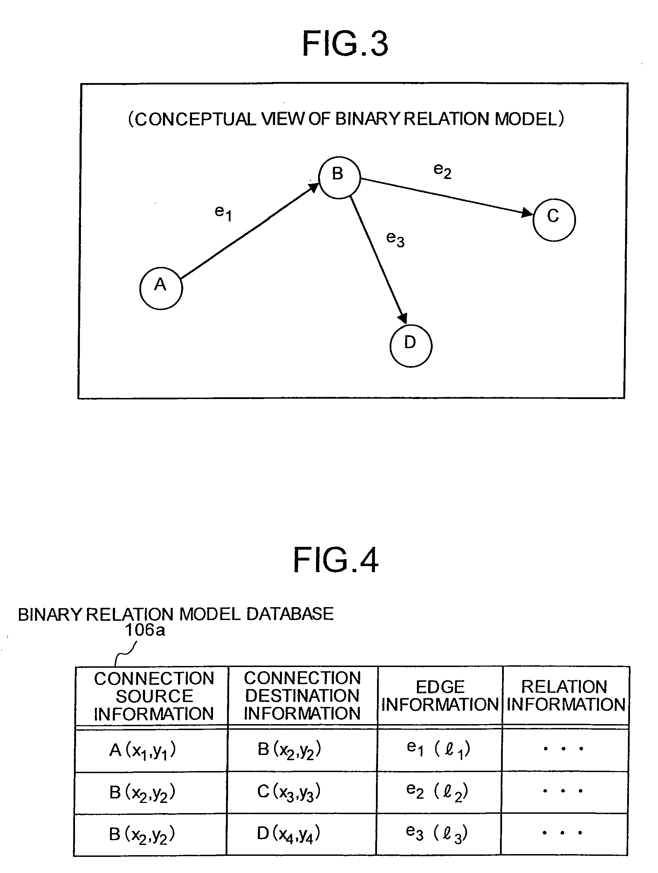

Among the constituent elements of the storage unit 106, the binary relation model database 106a is a database that stores binary relation models of pathway information. For instance, a binary relation model related to information pathway stored in the external database such as KEGG is created and stored in the binary relation model database 106a. Fig. 3 is a conceptual view of the binary relation model. In the example shown in Fig. 3, nodes A to D are connected to one another by edges $e_1$ to $e_3$. As described above, the pathway information can be defined by the binary relation model configured by nodes and edges.

Fig. 4 is one example of information stored in the binary relation model database 106a. As shown in Fig. 4, the information stored in the binary relation model database 106a is configured by associating connection source information including a name, a coordinate, and the like of each node of the connection source, connection destination information including a name, a coordinate, and the like of each node of the connection destination, edge information including a name, a length (distance), and the like of each edge, relation information indicating a relation between connected nodes (formation, inhibition, acceleration, etc.), and the like to one another. The graph data 106b is a graph data storage unit that stores data on a drawn graph.

In Fig. 2, the communication control interface unit 104 exercises a communication control over a communication between the pathway information display apparatus 100 and the network 300 (or the communication device such as a router). Namely, the communication control interface unit 104 functions to communicate data with the other terminal through the communication line.

In Fig. 2, the input and output control interface unit 108 controls the input device 112 and the output device 114. As the output device 114, a monitor (including a home television set) or a speaker can be employed (hereinafter, the monitor will be explained as the output device). As the input device 112, a keyboard, a mouse, a microphone, and the like can be employed. The monitor in cooperation with the mouse realizes a pointing device function.

In Fig. 2, the control unit 102 includes an internal memory for storing control programs for OS (Operating System) and the like, programs that specify various processing procedures, and required data. The control unit 102 performs information processing for executing various processing. The control unit 102 functionally and conceptually includes a binary relation model creation unit 102a, an attractive force calculation unit 102b, a repulsive force calculation unit 102c, a repulsion field calculation unit 102d, a boundary determination unit 102e, a dynamic parameter control unit 102f, a graph drawing unit 102g, an energy calculation unit 102h, and a dummy node processing unit 102i.

Among these constituent elements of the control unit 102, the binary relation model creation unit 102a is binary relation model creation means for creating the binary relation model of the pathway information configured by nodes and edges. The attractive force calculation unit 102b is attractive force calculation means for calculating an attractive force generated between nodes connected to each other by edges. The repulsive force calculation unit 102c is repulsive force calculation means for calculating a repulsive force generated between the nodes within a certain distance. The repulsion field calculation unit 102d is repulsion field calculation means for setting a repulsion field for a preset peripheral region of the nodes and for calculating the repulsive force and the attractive force in the repulsion field.

The boundary determination unit 102e is boundary determination means for determining whether a coordinate position of each node falls within a preset drawing region, and that, if the node coordinate position is out of the drawing region, moving the node coordinate position to a boundary of the drawing region. The dynamic parameter control unit 102f is dynamic parameter control means for controlling the repulsion field of the nodes to be narrowed and the attractive force between the nodes to be strengthened before a preset time passes, and for controlling the repulsion field of the nodes to be widened, the attractive force between the nodes to be weakened, and the internode repulsive force to be strengthened after the preset time passes. By doing so, the dynamic parameter control unit 102f controls graph drawing so that relevant nodes are closer before the preset time passes and so that the closer nodes are dispersed after passage of the preset time.

The graph drawing unit 102g is graph drawing means for determining the coordinate position of each node based on the repulsive force calculated by the repulsive force calculation unit, the attractive force calculated by the attractive force calculation unit, and the repulsive force and attractive force of the repulsion field calculated by the repulsion field calculation unit by the spring layout, and drawing the nodes and the edges in the form of a graph. The energy calculation unit 102h is energy calculation means for calculating energy by calculating a sum of absolute values of synthetic vectors based on the nodes and edges drawn by the graph drawing unit.

The dummy node processing unit 102i is dummy node setting means for, if the edge connection destination of each node is an edge other than that connected to the node, setting a dummy node on the other edge. Processing performed by these respective units will be explained later in detail.

[SYSTEM PROCESSING]

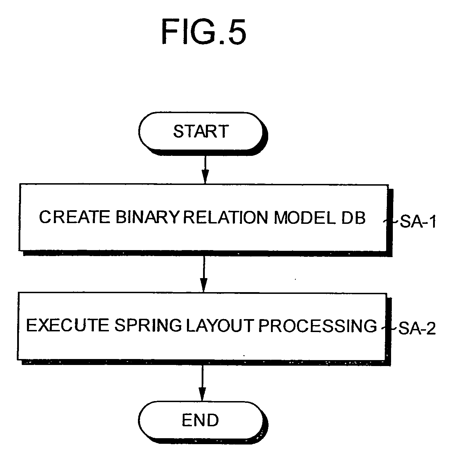

One example of processing of the apparatus according to this embodiment thus configured will be explained in detail with reference to Figs. 5 to 9.

[MAIN PROCESSING]

Main processing will be explained first in detail with reference to Fig. 5. Fig. 5 is a flowchart of one example of the main processing performed by the system according to this embodiment.

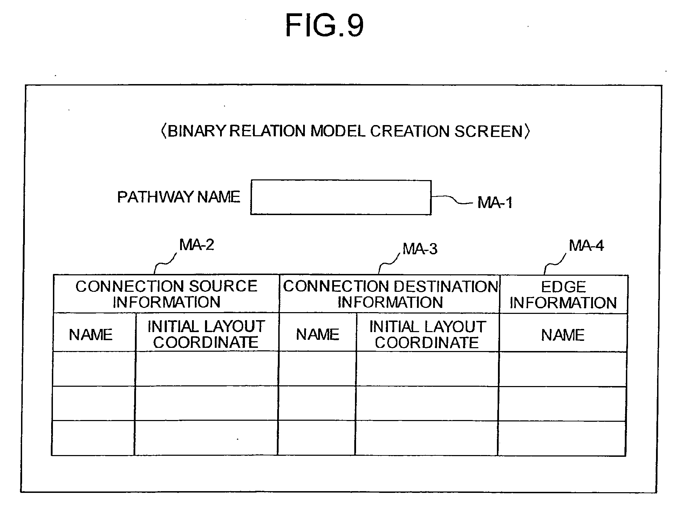

The pathway information display apparatus 100 creates the binary relation model from the pathway information by the processing performed by the binary relation model creation unit 102a, and stores the binary relation model in the binary relation model database 106a (at a step SA-1). Namely, the binary relation model creation unit 102a causes a user to select desired pathway information from among the pathway information stored in the database such as the conventional KEGG. The binary relation model creation unit 102a then automatically converts the binary relation model of the pathway information shown in Fig. 3 into a form shown in Fig. 4 to thereby create the binary relation model, and stores the binary relation model in the binary relation model database 106a. Alternatively, the binary relation model creation unit 102a can create the binary relation model by displaying a binary relation model creation screen as shown in Fig. 9 on the output device 114 and causing the user to input information on nodes and edges.

Fig. 9 is one example of the binary relation model creation screen displayed on the output device 114 of the pathway information display apparatus 100. As shown in Fig. 9, the binary relation model creation screen includes a pathway name input area MA-1, a connection source information input area MA-2, a connection destination information input area MA-3, and an edge information input area MA-4. If the user inputs desired information in these input areas through the input device 112, the binary relation model creation unit 102a stores the input information in a predetermined storage region of the binary relation model database 106a.

The control unit 102 executes a spring layout processing, to be explained later, based on the binary relation model database 106a (at a step SA-2). The main processing is thus finished.

[SPRING LAYOUT PROCESSING]

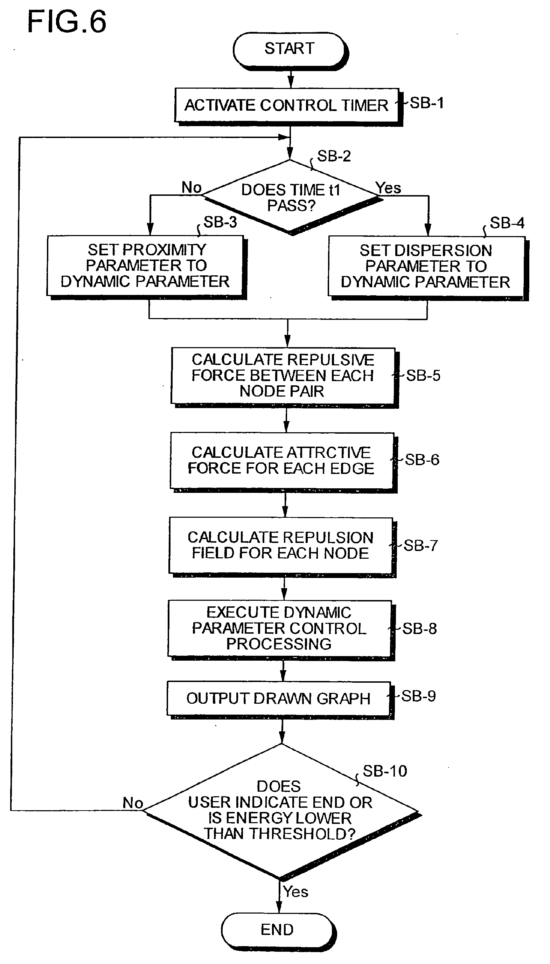

The spring layout processing will be explained in detail with reference to Fig. 6. Fig. 6 is a flowchart of one example of the spring layout processing performed by the system according to this embodiment.

The control unit 102 receives a system clock from the clock generator 110 and activates a control timer (at a step SB-1), and monitors whether a preset time t1 passes (at a step SB-2). The time t1 can be set based on a graph complexity (determined according to, for example, the number of nodes and edges), an energy value of an initial graph or the like.

If it is monitored that the time t1 does not pass at the step SB-2, a proximity parameter is set to a dynamic parameter (at a step SB-3). When the time t1 passes, a dispersion parameter is set to the dynamic parameter (at a step SB-4).

The repulsive force calculation unit 102c calculates and sets a repulsive force between each node pair within a certain distance (at a step SB-5). Namely, the repulsive force calculation unit 102c sets a stronger repulsive force between the nodes as the distance between them becomes shorter.

The attractive force calculation unit 102b then calculates and sets an attractive force between every node pair connected to each other by edges (at a step SB-6). Namely, the attractive force calculation unit 102b sets a stronger attractive force between the nodes as the distance between them becomes longer.

The repulsion field calculation unit 102d sets a repulsion field for the peripheral region of the nodes, calculates the repulsive force and the attractive force in the repulsion field, and secures a character region for labels of nodes and the like (at a step SB-7). Namely, the repulsion field calculation unit 102d calculates the repulsive force and the attractive force in the repulsion field by performing both or one of weakening of the attractive force for the node peripheral region (including not generating an attractive force) and strengthening of the repulsive force between each node pair located within the node peripheral region.

For instance, the repulsive force calculation unit 102d calculates the repulsive force and the attractive force of the repulsion field by setting, for example, the repulsion field as a 20% similar region to a node size from a center of the nodes, the attractive force to 95% of the distance, and the repulsive force to 200%, or by setting the repulsion field as a 400% similar region to the node size from the center of the nodes, the attractive force to 5% of the distance, and the repulsive force to 800%.

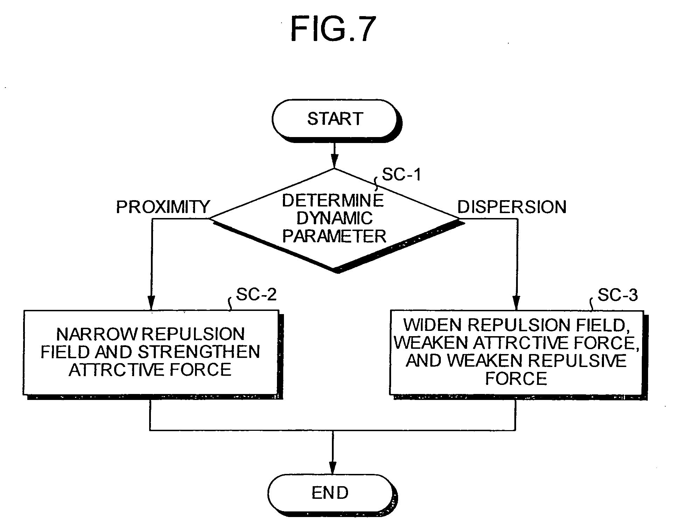

The dynamic parameter control unit 102f executes a dynamic parameter control processing shown in Fig. 7 (at a step SB-8).

Fig. 7 is a flowchart of one example of the dynamic parameter control processing according to the embodiment of the present invention. In Fig. 7, the dynamic parameter control unit 102f determines whether the set dynamic parameter is a proximity parameter or a dispersion parameter (at a step SC-1). If the dynamic parameter is the proximity parameter, the dynamic parameter control unit 102f controls the repulsion field to be narrowed and the attractive force to be strengthened (at a step SC-2). If the dynamic parameter is the dispersion parameter, the dynamic parameter control unit 102f controls the repulsion field to be widened, the attractive force to be weakened, and the repulsive force to be weakened (at a step SC-3). Namely, if the dynamic parameter is the proximity parameter, the related nodes are closer on the graph. If the dynamic parameter is the dispersion parameter, the related nodes are dispersed on the graph.

Referring back to Fig. 6, the graph drawing unit 102g determines the coordinate position of each node based on the repulsive force and attractive force between the node pair and the repulsive force and attractive force in the repulsion field by the spring layout, and draws the nodes and edges in the form of a graph. Namely, the graph drawing unit 102g repeatedly executes a spring layout step of finely moving the node according to the synthetic vectors of all forces acting on the node by an appropriate number of times (the movement continues as long as forces are present). According to the spring layout, a force is defined as a function between a layout state and a time and controlled to be weaker if the node is moved in a direction of the force. Accordingly, if the number of steps increases, the sum of the absolute values of the entire forces in the layout (which will be referred to as "energy") is reduced. That is, the repulsive force enables the nodes to be apart from each other and not to be overlapped with each other. If the nodes are apart from each other by a predetermined distance or more, the repulsive force is zero. The attractive force enables the nodes connected to each other by edges to be closer to each other. If the nodes are closer to each other by the predetermined distance or more, the attractive force is zero. In addition, the action of the repulsive force can particularly make the.nodes having a large overlap amount apart from each other.

The spring layout will be explained referring to specific examples. The following processing is executed first to all node pairs (ni, nj) within the binary relation model.

(1) If a distance d between the nodes ni and nj exceeds a predetermined distance, the next node pair is processed.
(2) The nodes ni and nj are moved in opposite directions to the nodes nj and ni, respectively, by a distance proportional to a force (A) and inversely proportional to a square of d.
(3) If a position of the moved ni or nj is deviated from the drawing region, the node ni or nj is moved to a nearest boundary of the drawing region by the processing performed by the boundary determination unit 102e.

The following processing is then executed for all relations r within the binary relation model.

(1) A start point node and an end point node of the relation r are assumed as ns and ne, respectively. A distance between ns and ne is assumed as d.

(2) If the d falls within a preset distance, the next relation is processed.

(3) The nodes ns and ne are moved in an ne direction and an ns direction, respectively, by a distance proportional to a force (B) and the distance d.

The graph drawing unit 102g outputs the drawn graph to the output device 114 (at a step SB-9).

Data on the graph drawn halfway can be stored in the graph data 106b, and the respective control units (102a to 102h) can appropriately refer to or store the data on the graph drawn halfway stored in the graph data 106b.

If the user indicates an end using the input device 112 referring to the output graph, if the entire energy calculated by the energy calculation unit 102h is lower than a preset threshold, or the energy is a minimum, the spring layout processing is finished (at a step SB-10). Otherwise, the processing returns to the step SB-2.

[DUMMY NODE PROCESSING]

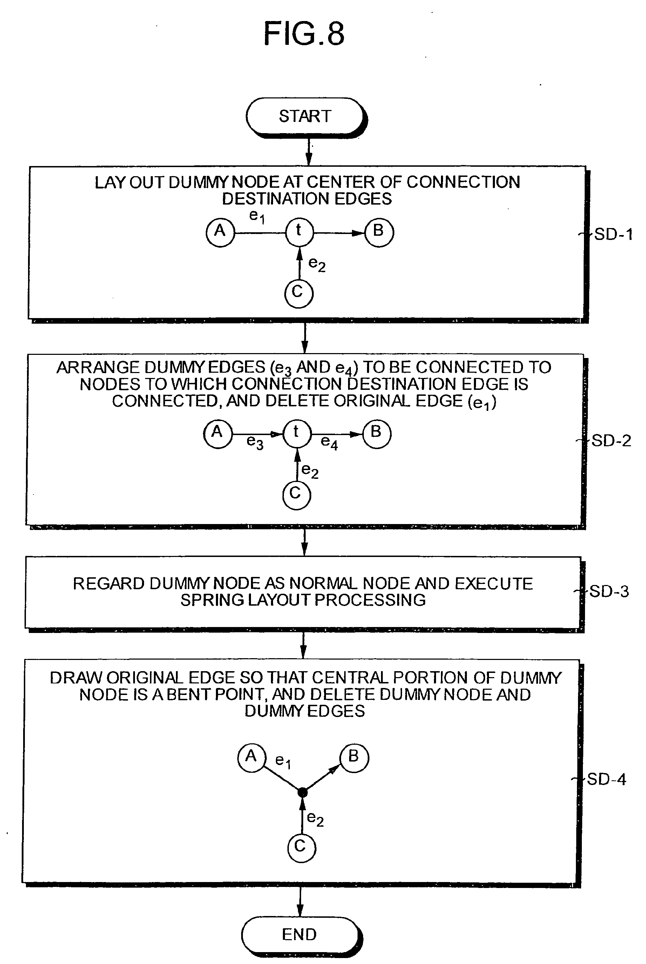

The dummy node processing will be explained in detail with reference to Fig. 8. Fig. 8 is a flowchart of one example of the dummy node processing performed by the system according to this embodiment.

During creation of the binary relation model, if a connection destination of the edge ($e_2$) is the edge ($e_1$), the dummy node processing unit 102i connects a dummy node (node t) in an appropriate form. Namely, the dummy node processing unit 102i puts the dummy node (node t) at a central portion or the like of the connection destination edge (e1) (at a step SD-1), arranges dummy edges (e3 and e4) to be connected to the node (node B) to which the connection destination edge (e1) is connected, and the original edge (e1) is deleted (at a step SD-2).

Regarding the dummy node as a normal node, the dummy node processing unit 102i calculates a layout (executes the spring layout) by the above-explained method (at a step SD-3).

The dummy node processing unit 102i draws the original edge (e1) so that a central portion of the dummy node is a bent point, and deletes the dummy node and the dummy edges (at a step SD-4). The dummy node processing is thus finished.

[OTHER EMBODIMENTS]

While the embodiments of the present invention have been explained above, variously modified embodiments other than the explained ones can be made without departing from the scope of the technical spirit of the appended claims.

Of the respective processing explained in the embodiments, all of or a part of the processing explained as being performed automatically can be performed manually, or all of or a part of the processing explained as being performed manually can be performed automatically in a known method.

Information including the processing procedure, the control procedure, specific names, parameters such as various kinds of registration data and search conditions, display examples, and database configurations shown in the specification or in the drawings can be optionally changed, unless otherwise specified.

The respective constituent elements of the pathway information display apparatus 100 are only functionally conceptual and not always required to be physically configured as shown in Fig. 2.

For instance, all of or a part of processing functions included by the respective servers of the pathway information display apparatus 100, particularly those executed by the control unit can be realized by the CPU (Central Processing Unit) and the programs interpreted and executed by the CPU. Alternatively, they can be realized as hardware based on wired logic. The programs are recorded in a recording medium to be explained later, and the programs are mechanically read by the pathway information display apparatus 100 according to need.

The various databases and the like (the binary relation model database 106a and the graph data 106b) stored in the storage unit 106 are storage means such as memory devices such as RAMs or ROMs, fixed disk devices such as hard disks, flexible disks, and optical disks.

They store the various programs, tables, files, databases, webpage files, and the like employed for various processing and for providing websites.

The pathway information display apparatus 100 can be realized by connecting an information processing apparatus such as an information processing terminal such as an existing personal computer or workstation to a peripheral such as a printer, a monitor, an image scanner or the like, and installing software (including programs, data, and the like) for realizing the method according to the present invention into the information processing apparatus.

Furthermore, a specific form of dispersion and integration of the pathway information display apparatus 100 is not limited to that shown in the drawings. All of or a part of the pathway information display apparatus 100 can be configured by being functionally or physically dispersed or integrated according to arbitrary units according to various loads or the like. For instance, the pathway information display apparatus 100 can be configured such that the respective databases serve as independent database apparatuses or a part of the processing thereof can be realized using CGI (Common Gateway Interface).

The programs according to the present invention can be stored in a computer readable recording medium. Examples of the "recording medium" include arbitrary "portable physical mediums" such as a flexible disk, a magnetooptical disk, a ROM, an EPROM, an EEPROM, a CD-ROM, an MO, and a DVD, arbitrary "fixed physical mediums" such as a ROM, a RAM, and a HD included in various computer systems, and "communication mediums" temporarily holding programs such as a communication line and a carrier wave used when the programs are transmitted through networks represented by a LAN, a WAN, and the Internet.

Furthermore, the "program" is a data processing method described in an arbitrary language by an arbitrary description method, and a form of the "program" is not limited to a source code or a binary code. The "program" is not always configured as a single program but can be configured to be distributed as a plurality of modules or libraries, or can fulfill the functions in cooperation with a separate program represented by OS (Operating System). As for specific configurations, reading procedures, installing procedures after reading, and the like for reading data from the recording medium by the respective devices shown in the embodiment, well-known configurations and procedures can be used, respectively.

The network 300 functions to connect the pathway information display apparatus 100 and the external system 200 to each other. For example, the network 300 can include any one of the Internet, the Intranet, a LAN (can be wired or wireless), a VAN, a personal computer communication network, a public telephone network (can be analog or digital), a dedicated line network (can be analog or digital), a CATV network, a portable line switched network or a portable packet switched network according to IMT2000, GSM, PDC/PDC-P or the like, a paging network, a local wireless network such as Bluetooth, a PHS network, a satellite communications network for CS, BS, ISDB or the like. Namely, the system according to the present invention can transmit and receive various pieces of data through an arbitrary network whether the network is wired or wireless.

As explained so far in detail, according to the present invention, a binary relation model of pathway information configured by nodes and edges is created. The pathway information includes information on genetic control pathways, metabolic pathways, signal-transfer pathways or the like. By mapping main information on a binary relation, the complicated pathway information can be simplified into a model and integrally expressed. Furthermore, a repulsive force generated between the nodes within a certain distance is calculated, and an attractive force generated between the nodes connected to each other by the edges is calculated. A coordinate position of each node is determined based on the repulsive force and the attractive force, and the nodes and the edges are drawn in the form of a graph. By doing so, even if the pathway information is medium or large-sized pathway information or complicated pathway information, a graph that is readable to some extent can be automatically drawn within a certain time.

If the layout is executed only by forces based on the repulsive force and the attractive force between each node pair, the nodes are often so close to each other, to make the edges and their labels difficult to distinguish from one another. According to the present invention, therefore, the repulsion field is set for a preset peripheral region of the nodes so as to prevent every node pair from being too close to each other. That is, according to the present invention, the repulsion field is set for the preset peripheral region of the nodes and the repulsive force and the attractive force are then calculated. Based on the calculated repulsive force and attractive force, and the repulsive force and attractive force in the repulsion field, the coordinate position of each node is determined by the spring layout, the nodes and the edges are drawn in the form of a graph, and the pathway information is displayed as a graph using the binary relation model. Therefore, overlapping of the nodes and the edges themselves and their labels is avoided, thereby further improving readability of the graph.

According to the present invention, the pathway information display apparatus, the pathway information display method, the program, and the recording medium configured as follows can be provided. The repulsion field is calculated by performing both of or one of weakening of the attractive force for the peripheral regions of the nodes (including not generating the attractive force) and strengthening of the repulsive force between every node pair located within the node peripheral region. Based on the forces, coordinate positions of the entire nodes are determined so that an entire force (e.g., an energy value that is the sum of absolute values of synthetic vectors for all edges) can realize the system in a minimum energy state by the spring layout. The nodes and the edges are then drawn in the form of a graph.

According to the present invention, it is determined whether a coordinate position of each node falls within a preset drawing region. If the node coordinate position is out of the drawing region, the node coordinate position is moved to a boundary of the drawing region. Therefore, the pathway information display apparatus, the pathway information display method, the program, and the recording medium capable of always drawing the pathway information within the drawing region can be provided.

According to the present invention, the node repulsion field is controlled to be narrowed and the attractive force generated between the nodes is controlled to be strengthened before a preset time passes, and the node repulsion field is controlled to be widened. In addition, the attractive force generated between the nodes is controlled to be weakened, and the repulsive force generated between the nodes is controlled to be strengthened after the preset time passes. It is thereby possible to provide the pathway information display apparatus, the pathway information display method, the program, and the recording medium capable of controlling the graph to be drawn so that the relevant nodes are closer to each other before the time passes and so that the closer nodes are dispersed after the time passes.

According to the present invention, the energy is calculated by calculating a sum of absolute values of synthetic vectors based on the drawn nodes and edges. The dynamic parameter control is repeatedly executed until the calculated energy is lower than a preset value, or until the energy becomes minimum. It is thereby possible to provide the pathway information display apparatus, the pathway information display method, the program, and the recording medium capable of calculating the layout so as to realize the system in a low energy state to a certain extent or in a minimum energy state.

Furthermore, according to the present invention, if a connection destination of an edge for one of the nodes is another one of the edges, a dummy node on another edge is set, and the graph is drawn while using a portion of the dummy node as a bent point. It is, therefore, possible to provide the pathway information display apparatus, the pathway information display method, the program, and the recording medium capable of improving the readability of the graph if the edge is connected to another edge.

(II) An embodiment of a directed graph layout apparatus, a directed graph layout method, a program, and a recording medium according to the present invention will be explained hereinafter in detail with reference to the accompanying drawings. Note that the invention is not limited by this embodiment.

In the following embodiment, an instance that the present invention is applied to a bioinformatics system that draws a graph of pathway information such as a genetic control pathway, a metabolic pathway, or a signal-transfer pathway will be explained. However, the invention is not limited to the instance, and can be similarly applied to a system that draws a graph of pathway information in every field.

[OUTLINE OF THE PRESENT INVENTION]

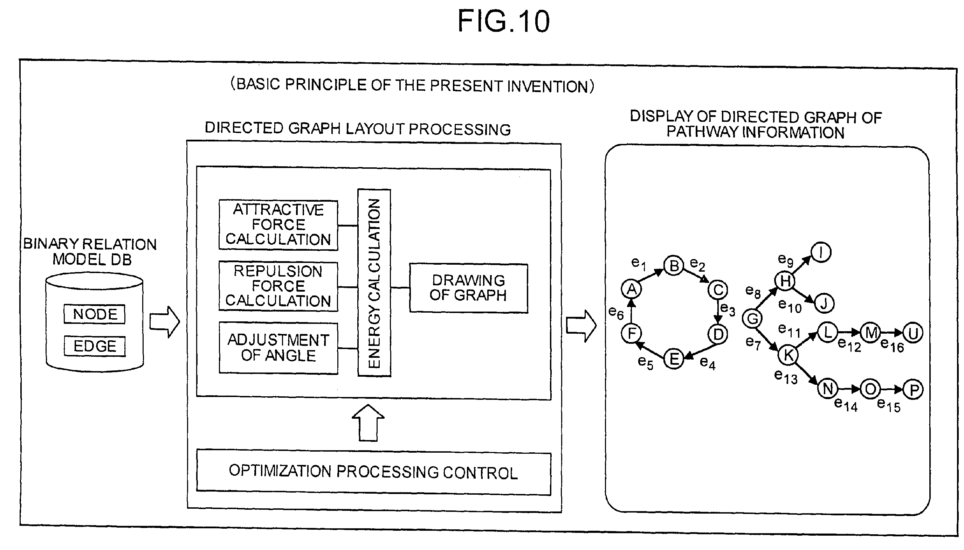

The outline of the basic principle of the present invention will be explained first, and a configuration, processing and the like of the invention will be explained next in detail. Fig. 10 is a principle block diagram of the basic principle of the present invention.

According to the present invention, a binary relation model of pathway information configured by nodes and edges is created first to thereby construct a binary relation model database. The "nodes" express matters or phenomena. The "edges" express various internode relations. The pathway includes a node serving as a start point and a node or an edge serving as an end point, and expresses a binary relation from the start point to the terminal point. For instance, a node expresses either a matter such as a gene, a protein, an amino acid, an enzyme, a coenzyme or the like, or a phenomenon such as an enzyme reaction or a metabolic reaction. An edge expresses a relation between the start point and the end point such as formation, inhibition or bonding. Normally, nodes and edges are expressed in different forms according to types of pathways.

A spring layout system or the like is then executed to nodes (A to U in Fig. 10) and edges ($e_1$ to $e_{15}$) defined in a binary relation model database to thereby automatically draw a graph. The "spring layout" is a layout called "force-directed layout" among ordinary undirected graph drawing schemes. Nodes are replaced by rings and edges are replaced by springs, and a layout is calculated so that forces of the springs (a repulsive force and an attractive force) acting on the rings can realize a minimum energy state of the system. Namely, according to the present invention, a repulsive force and an attractive force between nodes are calculated according to a distance between the nodes. Node coordinate positions are determined based on the calculated forces, and the nodes and the edges are drawn in the form of a graph. The "repulsive force" is a force generated between every pair of nodes present within a certain distance, and the nodes are dispersed and overlaps of nodes are removed by the repulsive force. The "attractive force" is a force generated between every pair of nodes present within the certain distance. The nodes connected to each other by edges by the attractive force are closer to each other. Therefore, relevant nodes can be made closer and easily discriminated from irrelevant nodes.

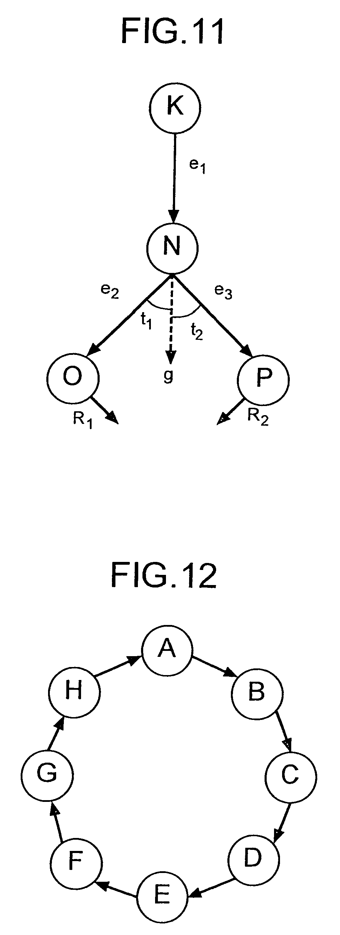

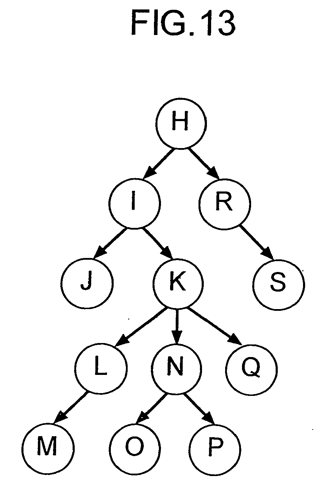

The spring layout is an undirected graph layout scheme that does not consider directivities of respective edges. For instance, if a directed graph (nodes A to H) such as the metabolic pathway on which a forward path is present as shown in Fig. 12 or a directed graph (nodes H to S) such as the signal-transfer pathway on which a cyclic path is not present as shown in Fig. 13 is to be automatically laid out, the layout is performed without any considerations to the directivities of the respective edges.

According to the present invention, therefore, the respective nodes are laid out by the spring layout or the like, and angles of the respective edges are then adjusted according to the directions of the edges, thereby drawing a graph. Namely, according to the present invention, an angle correcting force that is a force for making directions of edges connected to each node uniform is generated, thereby controlling layout of the nodes.

Fig. 11 depicts a concept of an angle adjustment using the angle correcting force according to the present invention. According to the invention, respective directed edges ($e_1$, $e_2$, and $e_3$ shown in Fig. 11) connected to a node (a node N in Fig. 11) are set as unit vectors, and a synthetic vector (g in Fig. 11) of all the unit vectors is generated.

According to the present invention, angle correcting forces ($R_1$ and $R_2$ in Fig. 11) are generated for connection destination nodes (nodes O and P in Fig. 11) of the directed edges in a direction in which angles ($t_1$ and $t_2$ in Fig. 11) between the synthetic vector (g) and the unit vectors ($e_2$ and $e_3$) are made smaller and in a direction perpendicular to the unit vectors, respectively. The angle correcting forces can be a constant value or changed according to the respective angles (e.g., numeric values incremented when the angles are larger).

Alternatively, the unit vector as each directed edge can be multiplied by a coefficient according to a type of the directed edge, and each synthetic vector can be generated using the unit vector multiplied by the coefficient. By doing so, a weight can be given to the directivity of the directed edge according to the type of the directed edge.

According to the present invention, the angle correcting forces of all the nodes are summed up to calculate energy, and coordinate positions of the nodes are changed so as to optimize the calculated energy. Alternatively, the energy can be calculated by generally adding up other forces (e.g., attractive forces and repulsive forces) calculated by the spring layout or the like in the entire graph. Namely, a sum of absolute values of the forces can be calculated as the energy, and layout of the respective nodes can be determined so as to minimize the energy by an ordinary optimization scheme (e.g., genetic algorithm or simulated annealing). When the angle adjustment according to the present invention is made, all the directed edges are to be overlaid on a certain line (synthetic vector). However, for example, by utilizing a force-directed layout scheme such as the spring layout together with the invention, the repulsive force generated between nodes closer to each other to some extent is stronger, and the nodes repel each other. It is, therefore, possible to prevent different edges from being completely overlaid.

As the present invention is used, in the directed graph (nodes A to H) such as the metabolic pathway on which the forward path is present as shown in Fig. 12, the respective nodes are arranged on a circle by forces for causing the directed edges to be aligned on a line. In the directed graph (nodes H to S) such as the signal-transfer pathway on which the cyclic path is not present as shown in Fig. 13, directions of the directed edges are made uniform to some extent around the respective nodes. The directions of the edges are thereby made uniform as a whole.

[SYSTEM CONFIGURATION]

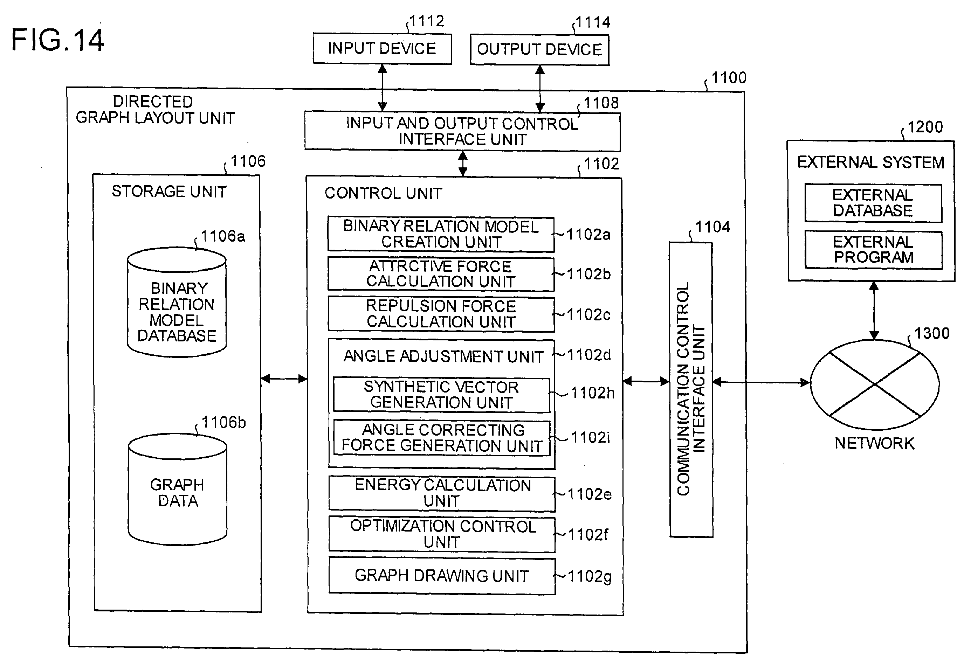

A configuration of a system to which the present invention is applied will be explained. Fig. 14 is a block diagram of one example of the configuration of the system to which the present invention is applied. In Fig. 14, only elements related to the invention are conceptually shown among all constituent elements of the system. The system is roughly configured such that a directed graph layout apparatus 1100 and an external system 1200 are communicably connected to each other through a network 1300. The external system 1200 provides pathway information and the like including external databases related to genetic control pathways, metabolic pathways, and information transduction pathways, external programs and the like for a homology search, and the like.

In Fig. 14, the network 1300, which functions to connect the directed graph layout apparatus 1100 to the external system 1200, is, for example, the Internet.

In Fig. 14, the external system 1200 is connected to the directed graph layout apparatus 1100 through the network 1300. The external system 1200 has a function to provide users with websites for executing the external databases related to the pathway information and the like and the external programs for the homology search, a motif search, and the like.

The external system 1200 can be configured as a WEB server, an ASP server or the like, and a hardware configuration thereof can be configured by an information processing apparatus such as a commercially available workstation or personal computer as well as their additional apparatuses. Functions of the external system 1200 are realized by a CPU, a disk device, a memory device, an input device, an output device, a communication control apparatus, and the like as well as programs for controlling these apparatuses and the like.

In Fig. 14, the directed graph layout apparatus 1100 generally includes a control unit 1102 such as a CPU, a communication control interface unit 1104, an input and output control interface unit 1108, and a storage unit 1106. The communication control interface unit 1102 integrally controls entirety of the directed graph layout apparatus 1100. The communication control interface unit 1104 is connected to a communication device (not shown), e.g., a router connected to a communication line or the like. The input and output control interface unit 1108 is connected to the input device 1112 and the output device 1114. The storage unit 1106 stores various databases and tables (a binary relation model database 1106a and a graph data 1106b). These constituent elements are communicably connected to the network 1300. Furthermore, the directed graph layout apparatus 1100 is communicably connected to the network 1300 through the communication device such as a router and a wired or wireless communication line such as a dedicated line.

The various databases and tables (the binary relation model database 1106a and the graph data 1106b) stored in the storage unit 1106 are storage means such as a fixed disk device and the like, and store various programs, tables, databases, webpage files, and the like used for various processing.

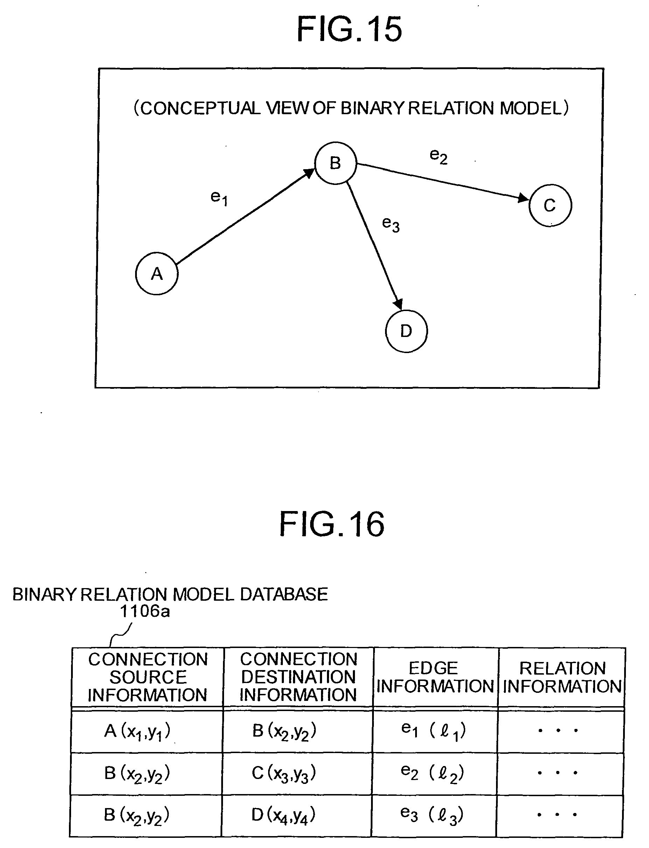

Among the constituent elements of the storage unit 1106, the binary relation model database 1106a is a database that stores binary relation models of pathway information. For instance, a binary relation model related to pathway information stored in the external database such as KEGG is created and stored in the binary relation model database 1106a. Fig. 15 is a conceptual view of the binary relation model. In the example shown in Fig. 15, nodes A to D are connected to one another by edges $e_1$ to $e_3$. As described above, the pathway information can be defined by the binary relation model configured by nodes and edges.

Fig. 16 is one example of information stored in the binary relation model database 1106a. As shown in Fig. 16, the information stored in the binary relation model database 1106a is configured by associating connection source information including a name, a coordinate, and the like of each node of the connection source, connection destination information including a name, a coordinate, and the like of each node of the connection destination, edge information including a name, a length (distance), and the like of each edge, relation information indicating a type of a relation between connected nodes (formation, inhibition, acceleration, normal system, or abnormal system), and the like to one another. The graph data 1106b is graph data storage means for storing data on a drawn graph.

In Fig. 14, the communication control interface unit 1104 exercises a communication control over a communication between the directed graph layout apparatus 1100 and the network 1300 (or the communication device such as a router). Namely, the communication control interface unit 1104 functions to communicate data with the other terminal through the communication line.

In Fig. 14, the input and output control interface unit 1108 controls the input device 1112 and the output device 1114. As the output device 1114, a monitor (including a home television set) or a speaker can be employed (hereinafter, the monitor will be explained as the output device). As the input device 1112, a keyboard, a mouse, a microphone, and the like can be employed. The monitor in cooperation with the mouse realizes a pointing device function.

In Fig. 14, the control unit 1102 includes an internal memory for storing control programs for OS (Operating System) and the like, programs that specify various processing procedures, and required data. The control unit 1102 performs information processing for executing various processing. The control unit 1102 functionally and conceptually includes a binary relation model creation unit 1102a, an attractive force calculation unit 1102b, a repulsive force calculation unit 1102c, an angle adjustment unit 1102d, an energy calculation unit 1102e, an optimization control unit 1102f, a graph drawing unit 1102g, a synthetic vector generation unit 1102h, and an angle correcting force generation unit 1102i.

Among these constituent elements of the control unit 1102, the binary relation model creation unit 1102a is binary relation model creation means for creating the binary relation model of the pathway information configured by nodes and edges. The attractive force calculation unit 1102b is attractive force calculation means for calculating an attractive force generated between nodes connected to each other by edges. The repulsive force calculation unit 1102c is repulsive force calculation means for calculating a repulsive force generated between the nodes within a certain distance. The angle adjustment unit 1102d is angle adjustment means for adjusting an angle of each edge according to the direction of the edge.

The angle adjustment unit 1102d includes a synthetic vector generation unit 1102h and the angle correcting force generation unit 1102i. The synthetic vector generation unit 1102h is synthetic vector generation means for generating a synthetic vector of all the unit vectors that are the directed edges connected to each node. The angle correcting force generation unit 1102i is angle correcting force generation means for generating an angle correcting force for a connection destination node of each directed edge in a direction in which the synthetic vector generated by the synthetic vector generation unit and each unit vector is made smaller and in a direction perpendicular to the unit vector.

The energy calculation unit 1102e is energy calculation means for calculating energy by calculating a sum of at least angle correcting forces, repulsive forces, or attractive forces for all nodes. The optimization control unit 1102f is optimization control means for changing a coordinate position of each node so as to optimize the energy calculated by the energy calculation unit. The graph drawing unit 1102g is graph drawing means for determining the coordinate position of each node based on the repulsive force, the attractive force, the angle correcting force and the like by the spring layout, and drawing the nodes and the directed edges in the form of a directed graph. Processing performed by these respective units will be explained later in detail.

[SYSTEM PROCESSING]

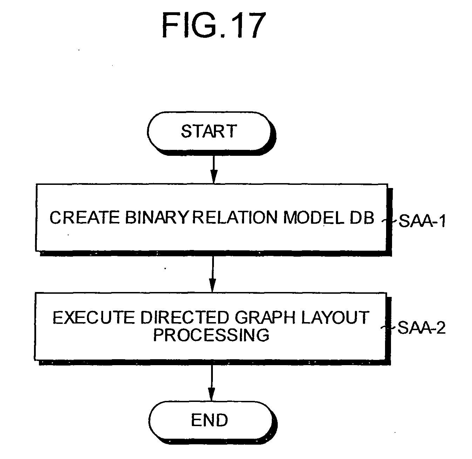

One example of processing of the apparatus according to this embodiment thus configured will be explained in detail with reference to Figs. 17 to 21.

[MAIN PROCESSING]

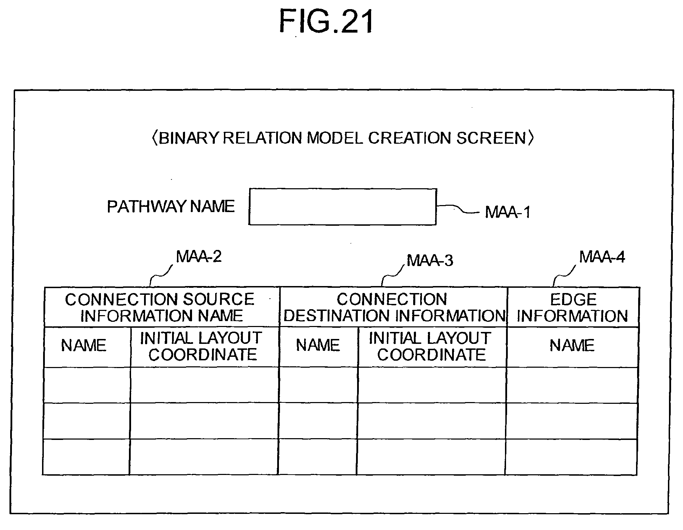

Main processing will be explained first in detail with reference to Figs. 17 and 21. Fig. 17 is a flowchart of one example of the main processing performed by the system according to this embodiment.

The directed graph layout apparatus 1100 creates the binary relation model from the pathway information by the processing performed by the binary relation model creation unit 1102a, and stores the binary relation model in the binary relation model database 1106a (at a step SAA-1). Namely, the binary relation model creation unit 1102a causes a user to select desired pathway information from among the pathway information stored in the database such as the conventional KEGG. The binary relation model creation unit 1102a then automatically converts the binary relation model of the pathway information shown in Fig. 15 into a form shown in Fig. 16 to thereby create the binary relation model, and stores the binary relation model in the binary relation model database 1106a. Alternatively, the binary relation model creation unit 1102a can create the binary relation model by displaying a binary relation model creation screen as shown in Fig. 21 on the output device 1114 and causing the user to input information on nodes and edges.

Fig. 21 is one example of the binary relation model creation screen displayed on the output device 1114 of the directed graph layout apparatus 1100. As shown in Fig. 21, the binary relation model creation screen includes a pathway name input area MAA-1, a connection source information input area MAA-2, a connection destination information input area MAA-3, and an edge information input area MAA-4. If the user inputs desired information in these input areas through the input device 1112, the binary relation model creation unit 1102a stores the input information in a predetermined storage region of the binary relation model database 1106a.

The control unit 1102 executes a directed graph layout processing, to be explained later, based on the binary relation model database 1106a (at a step SAA-2). The main processing is thus finished.

[DIRECTED GRAPH LAYOUT PROCESSING]

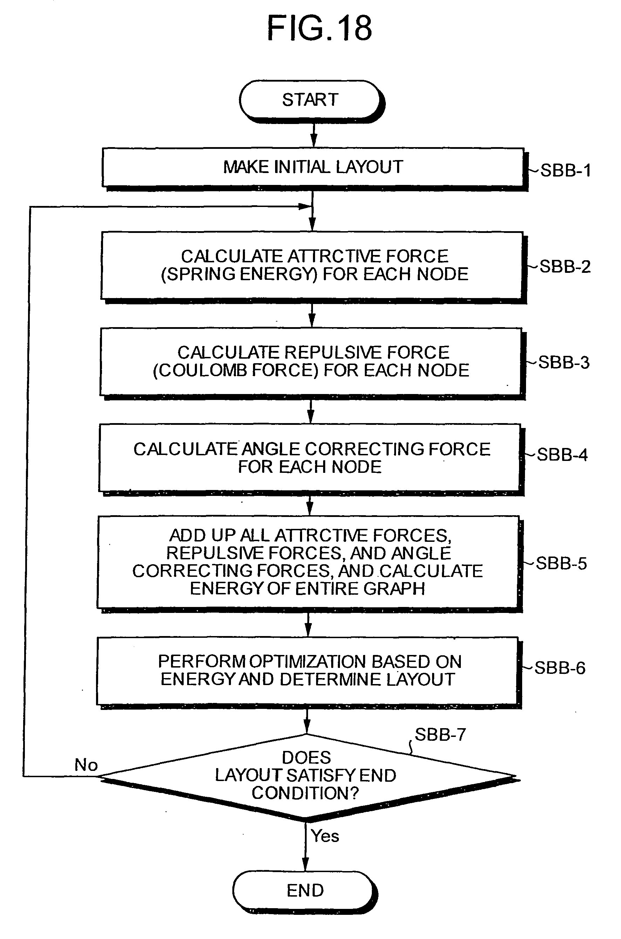

The directed graph layout processing will be explained in detail with reference to Figs. 18 to 20. Fig. 18 is a flowchart of one example of the directed graph layout processing performed by the system according to this embodiment.

It is assumed first that a directed graph is stored in G(N, E) in the binary relation model database 1106a.

Symbol N denotes a node set and is expressed by the following equation:

$$N = \{n_1, \ n_2, \ \ldots, \ n_n\}.$$

Symbol E denotes an edge set and is expressed by the following equation:

$$E = \{e_1(ns_1, \ nd_1), \ e_2(ns_2, \ nd_2), \ldots, \ e_n(ns_n, \ nd_n)\}$$

(where ns denotes a connection source node, and nd denotes a connection destination node.).

It is assumed that the directed graph is initially laid out ($c_0$) at random or at an initial coordinate position stored in the binary relation model database 1106a (at a step SBB-1). If so, a present layout position ($c_c$) is $c_0$.

The directed graph layout apparatus 1100 calculates the attractive force for each element $n_i$ in the node set N by the processing performed by the attractive force calculation unit 1102b (at a step SBB-2). The attractive force calculation unit 1102b calculates a spring energy (a force for causing edges to attract each other) for all edges $e_j$ connected to the node $n_i$ as expressed by the following equation, and calculates a sum of the spring energies.

$$|K \times (L-l)|$$

(where K is a coefficient of spring, L is a spring natural length constant, l is a distance between the node $n_i$ and a node ahead of the node $n_i$ connected to each other by the edge $e_j$)

The directed graph layout apparatus 1100 calculates the repulsive force for each element $n_i$ in the node set N by the processing performed by the repulsive force calculation unit 1102c (at a step SBB-3). For instance, the repulsive force calculation unit 1102c calculates a Coulomb force (a force for causing the nodes to repel each other) between every node pair as expressed by the following equation, and calculates a sum of the Coulomb forces.

$$C/d$$

(where C is a coefficient of the Coulomb force and d is an internode distance)

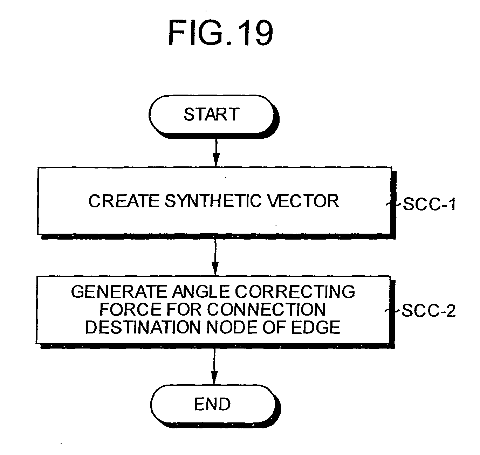

The directed graph layout apparatus 1100 calculates the angle correcting force that is a force for making the directions of the edges connected to each node uniform by the processing performed by the angle adjustment unit 1102d (at a step SBB-4). Fig. 19 is a flowchart of one example of the angle correcting force calculation processing performed by the angle adjustment unit 1102d. As shown in Fig. 19, the angle adjustment unit 1102d generates the synthetic vector of all unit vectors that are the respective directed edges $e_j$ connected to the node $n_i$ (at a step SCC-1).

The angle adjustment unit 1102d generates the angle correcting force for the connection destination node of each directed edge in a direction in which the synthetic vector (g), each unit vector, and an angle (t) in radians are made smaller and in a direction perpendicular to the unit vector as expressed by the following equation (at a step SCC-2).

$(\tan(t/2))^2$

Referring back to Fig. 18, the directed graph layout apparatus 1100 calculates a sum of attractive forces, repulsive forces, and angle correcting forces for all nodes and calculates energy of the entire graph by the processing performed by the energy calculation unit 1102e (at a step SBB-5) .

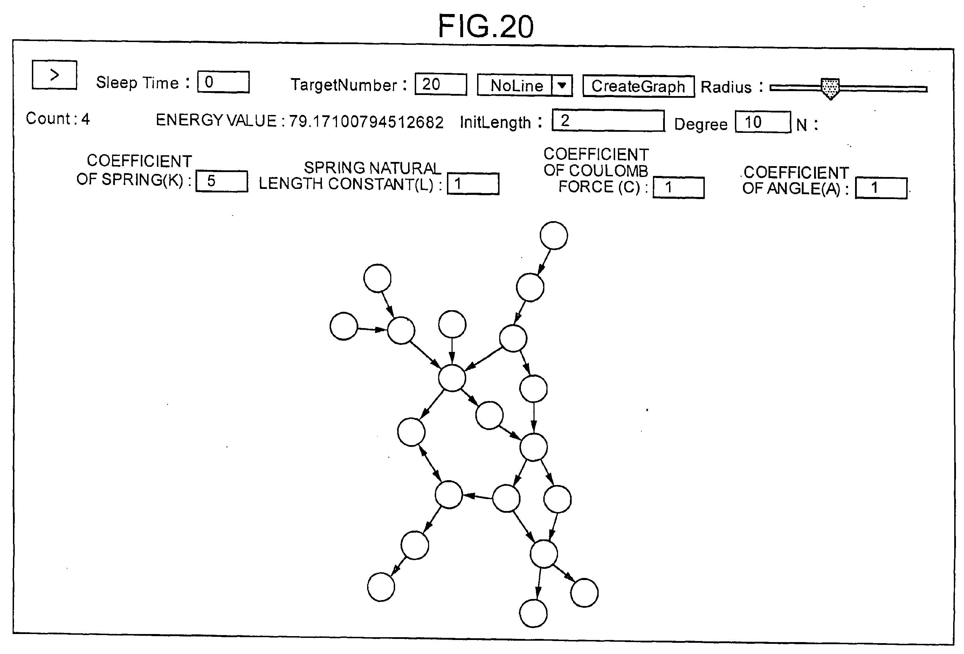

The directed graph layout apparatus 1100 changes the coordinate position of each node so as to optimize the calculated energy by the processing performed by the optimization control unit 1102f (at a step SBB-6). At this moment, the other forces (e.g., attractive forces and repulsive forces) calculated by the spring layout or the like are all added up in the entire graph and a sum of absolute values of the forces is calculated as the energy. In addition, the layout of the respective nodes is determined so that the energy is minimized by the ordinary optimization scheme (e.g., the genetic algorithm or the simulated annealing). The directed graph layout apparatus 1100 outputs the directed graph to the output device 1114 by the processing performed by the graph drawing unit 1102g. Fig. 20 is one example of a directed graph output screen displayed on the output device 1114 of the directed graph layout apparatus 1100.

The directed graph layout apparatus 1100 determines whether the created directed graph satisfies a preset end condition (e.g., whether the energy is lower than a preset threshold, whether the user designates the end, or whether the number of times accounts for a restricted number of times) by the processing performed by the optimization control unit 1102f (at a step SBB-7). If the created directed graph does not satisfy the end condition, the processing returns to the step SBB-2. The directed graph layout processing is thus finished.

[OTHER EMBODIMENTS]

While the embodiments of the present invention have been explained above, variously modified embodiments other than the explained ones can be made without departing from the scope of the technical spirit of the appended claims.

For example, the instance that the directed graph layout apparatus 1100 performs processing in a standalone form has been explained. Alternatively, the system can be configured such that the directed graph layout apparatus 1100 performs a processing in response to a client terminal configured by a different housing from the directed graph layout apparatus 1100, and returns a processing result to the client terminal.

Of the respective processing explained in the embodiments, all of or a part of the processing explained as being performed automatically can be performed manually, or all of or a part of the processing explained as being performed manually can be performed automatically in a known method.

Information including the processing procedure, the control procedure, specific names, parameters such as various kinds of registration data and search conditions, display examples, and database configurations shown in the specification or in the drawings can be optionally changed, unless otherwise specified.

The respective constituent elements of the directed graph layout apparatus 1100 are only functionally conceptual and not always required to be physically configured as shown in the drawings.

For instance, all of or a part of processing functions included by the respective servers of the directed graph layout apparatus 1100, particularly those executed by the control unit 1102 can be realized by the CPU (Central Processing Unit) and the programs interpreted and executed by the CPU. Alternatively, they can be realized as hardware based on wired logic. The programs are recorded in a recording medium to be explained later. The programs are mechanically read by the directed graph layout apparatus 1100 according to need.

That is, a computer program for transmitting a command to the CPU in cooperation with OS (Operating System) and for performing various processing is recorded in the storage unit 1106 such as the ROM or HD, or the like. This computer program is executed by being loaded to the RAM or the like, and the computer program and the CPU constitute the control unit. Alternatively, this computer program can be recorded in an application program server connected to the directed graph layout apparatus 1100 through an arbitrary network. All of or a part of the computer program can be downloaded according to need.

The programs according to the present invention can be stored in a computer readable recording medium. Examples of the "recording medium" include arbitrary "portable physical mediums" such as a flexible disk, a magnetooptical disk, a ROM, an EPROM, an EEPROM, a CD-ROM, an MO, and a DVD, arbitrary "fixed physical mediums" such as a ROM, a RAM, and a HD included in various computer systems, and "communication mediums" temporarily holding programs such as a communication line and a carrier wave used when the programs are transmitted through networks represented by a LAN, a WAN, and the Internet.

Furthermore, the "program" is a data processing method described in an arbitrary language by an arbitrary description method, and a form of the "program" is not limited to a source code or a binary code. The "program" is not always configured as a single program but can be configured to be distributed as a plurality of modules or libraries, or can fulfill the functions in cooperation with a separate program represented by OS (Operating System). As for specific configurations, reading procedures, installing procedures after reading, and the like for reading data from the recording medium by the respective devices shown in the embodiment, well-known configurations and procedures can be used, respectively.

The various databases (the binary relation model database 1106a and the graph data 1106b) stored in the storage unit 1106 are storage means such memory devices such as RAMs or ROMs, fixed disk devices such as hard disks, flexible disks, and optical disks. They store the various programs, tables, files, databases, webpage files, and the like employed for various processing and for providing websites.

The directed graph layout apparatus 1100 can be realized by connecting an information processing apparatus such as an information processing terminal such as an existing personal computer or workstation to a peripheral such as a printer, a monitor, an image scanner or the like, and installing software (including programs, data, and the like) for realizing the method according to the present invention into the information processing apparatus.

Furthermore, a specific form of dispersion and integration of the directed graph layout apparatus 1100 is not limited to that shown in the drawings. All of or a part of the directed graph layout apparatus 1100 can be configured by being functionally or physically dispersed or integrated according to arbitrary units according to various loads or the like. For instance, the directed graph layout apparatus 1100 can be configured such that the respective databases serve as independent database apparatuses or a part of the processing thereof can be realized using CGI (Common Gateway Interface).

The network 1300 functions to connect the directed graph layout apparatus 1100 and the external system 1200 to each other. For example, the network 1300 can include any one of the Internet, the Intranet, a LAN (can be wired or wireless), a VAN, a personal computer communication network, a public telephone network (can be analog or digital), a dedicated line network (can be analog or digital), a CATV network, a portable line switched network or a portable packet switched network according to IMT2000, GSM, PDC/PDC-P or the like, a paging network, a local wireless network such as Bluetooth, a PHS network, a satellite communications network for CS, BS, ISDB or the like. Namely, the system according to the present invention can transmit and receive various pieces of data through an arbitrary network whether the network is wired or wireless.

As explained so far in detail, according to the present invention, a synthetic vector of all unit vectors is generated, with the directed edges connected to each of the nodes assumed as the unit vectors. An angle correcting force is generated for the each node serving as a connection destination node of the directed edges in a direction in which an angle between the generated synthetic vector and each of the unit vectors is smaller and in a direction perpendicular to the each unit vector. Energy is calculated by calculating a sum of the angle correcting forces for all the nodes, and a position of each of the nodes is changed so as to optimize the calculated energy. It is, therefore, possible to provide the directed graph layout apparatus, the directed graph layout method, the program, and the recording medium capable of automatically drawing a graph in which flows of edges of a directed graph are expressed.

According to the present invention, it is possible to provide the directed graph layout apparatus, the directed graph layout method, the program, and the recording medium capable of automatically laying out a directed graph in which a pathway on which a forward path is present and a pathway on which a cyclic path is not present are mixed so that the forward path part is closer to a circle and directions of edges coincide with one another on a part other than the forward path, and realizing the directed graph layout efficiently by a simple method.

According to the present invention, it is possible to provide the directed graph layout apparatus, the directed graph layout method, the program, and the recording medium capable of generally effectively using the drawing region, as compared with an instance of drawing a graph using the magnetic spring scheme.

According to the present invention, each unit vector of each of the directed edges is multiplied by a coefficient according to a type of the directed edge, and the synthetic vector is generated using the unit vectors each multiplied by the coefficient. Therefore, a weight can be given to the directivity of each directed edge according to the type of the directed edge. For instance, as types of the edges, a normal route (a route in a stationary state), an abnormal route (a route appearing under abnormal conditions), and the like are set, and a weight is given to the normal route. It is thereby possible to provide the directed graph layout apparatus, the directed graph layout method, the program, and the recording medium capable of laying out the graph so that the directivity of each edge is along the normal route.

According to the present invention, the angle correcting force is a preset constant. It is, therefore, possible to provide the directed graph layout apparatus, the directed graph layout method, the program, and the recording medium capable of obtaining the angle correcting force without making complicated calculations, and without need of lots of calculation time and calculation amount.

According to the present invention, the angle correcting force is changed according to the angle. It is, therefore, possible to provide the directed graph layout apparatus, the directed graph layout method, the program, and the recording medium capable of efficiently executing the optimization in the initial state.

According to the present invention, an attractive force generated between the nodes connected to each other by the edges is calculated, and a repulsive force generated between the nodes within a certain distance is calculated. The energy is calculated by calculating a sum of the attractive forces, the repulsive forces, and the angle correcting forces for all the nodes. When the angle adjustment is made, all the directed edges are to be overlaid on a certain line (synthetic vector). However, by utilizing a force-directed layout scheme such as the spring layout together with the present invention, the repulsive force generated between nodes closer to each other to some extent is stronger, and the nodes repel each other. It is, therefore, possible to provide the directed graph layout apparatus, the directed graph layout method, the program, and the recording medium capable of preventing different edges from being completely overlaid.

INDUSTRIAL APPLICABILITY

(I) As explained so far, the pathway information display apparatus, the pathway information display method, the program, and the recording medium can automatically draw a graph for a complicated pathway, or a medium or large-sized pathway.

The pathway information display apparatus, the pathway information display method, the program, and the recording medium according to the present invention are, therefore, remarkably useful in the bioinformatics field of drawing graphs for pathway information such as genetic control pathways, metabolic pathways, and signal-transfer pathways.

The present invention can be widely carried out in many industrial fields, particularly in the fields of drugs, foods, cosmetic products, medical treatments, gene expression analyses, information processing, and the like, and therefore remarkably useful.

(II) Furthermore, the directed graph layout apparatus, the directed graph layout method, the program, and the recording medium can efficiently lay out a directed graph by a simple method if a pathway on which a forward path is present and a pathway on which a cyclic path is not present are mixed in the directed graph.

The directed graph layout apparatus, the directed graph layout method, the program, and the recording medium according to the present invention are, therefore, remarkably useful in the bioinformatics field of drawing graphs for pathway information such as genetic control pathways, metabolic pathways, and signal-transfer pathways.

The present invention can be widely carried out in many industrial fields, particularly in the fields of drugs, foods, cosmetic products, medical treatments, gene expression analyses, information processing, and the like, and therefore remarkably useful.

**Claims**

1. A pathway information display apparatus comprising:

   a binary relation model creation unit of creating a binary relation model of pathway information configured by nodes and edges;
   a repulsive force calculation unit of calculating a repulsive force generated between the nodes within a certain distance;
   an attractive force calculation unit of calculating an attractive force generated between the nodes connected to each other by the edges;
   a repulsion field calculation unit of calculating the repulsive force and the attractive force by setting a repulsion field for a preset peripheral region of the nodes; and
   a graph drawing unit of determining a coordinate position of each of the nodes based on the repulsive force calculated by the repulsive force calculation unit, the attractive force calculated by the attractive force calculation unit, and the repulsive force and the attractive force in the repulsion field calculated by the repulsion field calculation unit, using spring layout, and drawing the nodes and the edges in the form of a graph, wherein
   the pathway information is displayed as the graph using the binary relation model.

2. The pathway information display apparatus according to claim 1, wherein
   at the repulsive force calculation unit, the repulsive force and the attractive force in the repulsion field are calculated by performing both or one of weakening of the attractive force for the peripheral regions of the nodes and strengthening the repulsive force between the nodes located in the peripheral regions of the nodes.

3. The pathway information display apparatus according to claim 1, further comprising:

   a boundary determination unit of determining whether the coordinate position of the each node falls within a preset drawing region, and of, if the coordinate position is out of the drawing region, moving the coordinate position of the node to a boundary of the drawing region.

4. The pathway information display apparatus according to claim 1, further comprising:

   a dynamic parameter control unit of controlling the repulsion field generated between the nodes to be narrowed and the attractive force generated between the nodes to be strengthened before a preset time passes, controlling the repulsion field of the nodes to be widened, the attractive force generated between the nodes to be weakened, and the repulsive force generated between the nodes to be strengthened, and thereby controlling the graph to be drawn so that relevant nodes are closer to each other before the preset time passes and so that the closer nodes are dispersed from each other after passage of the preset time.

5. The pathway information display apparatus according to claim 4, further comprising:

   an energy calculation unit of calculating an energy by calculating a sum of absolute values of synthetic vectors based on the nodes and the edges drawn at the graph drawing unit, wherein
   the dynamic parameter control unit is repeatedly executed until the energy calculated at the energy calculation unit is lower than a preset value, or until the energy becomes minimum.

6. The pathway information display apparatus according to claim 1, further comprising:

   a dummy node setting unit of, if a connection destination of one of the edges for one of the nodes is another one of the edges, setting a dummy node on the another edge, wherein
   at the graph drawing unit, the graph is drawn while using a portion of the dummy node as a bent point.

7. A pathway information display method comprising:

   a binary relation model creation step of creating a binary relation model of pathway information configured by nodes and edges;
   a repulsive force calculation step of calculating a repulsive force generated between the nodes within a certain distance;
   an attractive force calculation step of calculating an attractive force generated between the nodes connected to each other by the edges;
   a repulsion field calculation step of calculating the repulsive force and the attractive force by setting a repulsion field for a preset peripheral region of the nodes; and
   a graph drawing step of determining a coordinate position of each of the nodes based on the repulsive force calculated by the repulsive force calculation step, the attractive force calculated by the attractive force calculation step, and the repulsive force and the attractive force in the repulsion field calculated by the repulsion field calculation step, using spring layout, and drawing the nodes and the edges in the form of a graph, wherein
   the pathway information is displayed as the graph using the binary relation model.

8. The pathway information display method according to claim 7, wherein
   at the repulsive force calculation step, the repulsive force and the attractive force in the repulsion field are calculated by performing both or one of weakening of the attractive force for the peripheral regions of the nodes and strengthening the repulsive force between the nodes located in the peripheral regions of the nodes.

9. The pathway information display method according to claim 7, further comprising:

   a boundary determination step of determining whether the coordinate position of the each node falls within a preset drawing region, and of, if the coordinate position is out of the drawing region, moving the coordinate position of the node to a boundary of the drawing region.

10. The pathway information display method according to claim 7, further comprising:

    a dynamic parameter control step of controlling the repulsion field generated between the nodes to be narrowed and the attractive force generated between the nodes to be strengthened before a preset time passes, controlling the repulsion field of the nodes to be widened, the attractive force generated between the nodes to be weakened, and the repulsive force generated between the nodes to be strengthened, and thereby controlling the graph to be drawn so that relevant nodes are closer to each other before the preset time passes and so that the closer nodes are dispersed from each other after passage of the preset time.

11. The pathway information display method according to claim 10, further comprising:

an energy calculation step of calculating an energy by calculating a sum of absolute values of synthetic vectors based on the nodes and the edges drawn at the graph drawing step, wherein
the dynamic parameter control step is repeatedly executed until the energy calculated at the energy calculation step is lower than a preset value, or until the energy becomes minimum.

12. The pathway information display method according to claim 7, further comprising:

a dummy node setting step of, if a connection destination of one of the edges for one of the nodes is another one of the edges, setting a dummy node on the another edge, wherein
at the graph drawing step, the graph is drawn while using a portion of the dummy node as a bent point.

13. A program capable of making a computer execute a pathway information display method comprising:

a binary relation model creation step of creating a binary relation model of pathway information configured by nodes and edges;
a repulsive force calculation step of calculating a repulsive force generated between the nodes within a certain distance;
an attractive force calculation step of calculating an attractive force generated between the nodes connected to each other by the edges;
a repulsion field calculation step of calculating the repulsive force and the attractive force by setting a repulsion field for a preset peripheral region of the nodes; and
a graph drawing step of determining a coordinate position of each of the nodes based on the repulsive force calculated by the repulsive force calculation step, the attractive force calculated by the attractive force calculation step, and the repulsive force and the attractive force in the repulsion field calculated by the repulsion field calculation step, using spring layout, and drawing the nodes and the edges in the form of a graph, wherein
the pathway information is displayed as the graph using the binary relation model.

14. The program according to claim 13, wherein
at the repulsive force calculation step, the repulsive force and the attractive force in the repulsion field are calculated by performing both or one of weakening of the attractive force for the peripheral regions of the nodes and strengthening the repulsive force between the nodes located in the peripheral regions of the nodes.

15. The program according to claim 13, further comprising:

a boundary determination step of determining whether the coordinate position of the each node falls within a preset drawing region, and of, if the coordinate position is out of the drawing region, moving the coordinate position of the node to a boundary of the drawing region.

16. The program according to claim 13, further comprising:

a dynamic parameter control step of controlling the repulsion field generated between the nodes to be narrowed and the attractive force generated between the nodes to be strengthened before a preset time passes, controlling the repulsion field of the nodes to be widened, the attractive force generated between the nodes to be weakened, and the repulsive force generated between the nodes to be strengthened, and thereby controlling the graph to be drawn so that relevant nodes are closer to each other before the preset time passes and so that the closer nodes are dispersed from each other after passage of the preset time.

17. The program according to claim 16, further comprising:

an energy calculation step of calculating an energy by calculating a sum of absolute values of synthetic vectors based on the nodes and the edges drawn at the graph drawing step, wherein
the dynamic parameter control step is repeatedly executed until the energy calculated at the energy calculation step is lower than a preset value, or until the energy becomes minimum.

**18.** The program according to claim 13, further comprising:

a dummy node setting step of, if a connection destination of one of the edges for one of the nodes is another one of the edges, setting a dummy node on the another edge, wherein
at the graph drawing step, the graph is drawn while using a portion of the dummy node as a bent point.

**19.** A computer readable recording medium having recorded therein the program according to any one of claims 13 to 18.

**20.** A directed graph layout apparatus for automatically laying out a directed graph configured by nodes and directed edges, comprising:

a synthetic vector generation unit of generating a synthetic vector of all unit vectors, the unit vectors being the directed edges connected to each of the nodes;
an angle correcting force generation unit of generating an angle correcting force for the each node serving as a connection destination node of the directed edges in a direction in which an angle between the synthetic vector generated at the synthetic vector generation unit and each of the unit vectors is smaller and in a direction perpendicular to the each unit vector;
an energy calculation unit of calculating an energy by calculating a sum of the angle correcting forces for all the nodes; and
an optimization control unit of changing a position of each of the nodes so as to optimize the energy calculated at the energy calculation unit.

**21.** The directed graph layout apparatus according to claim 20, further comprising:

a unit vector multiplication unit of multiplying the each unit vector of each of the directed edges by a coefficient according to a type of the each directed edge, wherein
at the synthetic vector generation unit, the synthetic vector is generated using the each unit vector multiplied by the coefficient at the unit vector multiplication unit.

**22.** The directed graph layout apparatus according to claim 20, wherein
the angle correcting force is a preset constant.

**23.** The directed graph layout apparatus according to claim 20, wherein
the angle correcting force is changed according to the angle.

**24.** The directed graph layout apparatus according to claim 20, further comprising:

an attractive force calculation unit of calculating an attractive force generated between the nodes connected to each other by the edges;
a repulsive force calculation unit of calculating a repulsive force generated between the nodes within a certain distance, wherein
at the energy calculation unit, the energy is calculated by calculating a sum of the attractive forces, the repulsive forces, and the angle correcting forces for all the nodes.

**25.** A directed graph layout method for automatically laying out a directed graph configured by nodes and directed edges, comprising:

a synthetic vector generation step of generating a synthetic vector of all unit vectors, the unit vectors being the directed edges connected to each of the nodes;
an angle correcting force generation step of generating an angle correcting force for the each node serving as a connection destination node of the directed edges in a direction in which an angle between the synthetic vector generated at the synthetic vector generation step and each of the unit vectors is smaller and in a direction perpendicular to the each unit vector;
an energy calculation step of calculating an energy by calculating a sum of the angle correcting forces for all the nodes; and
an optimization control step of changing a position of each of the nodes so as to optimize the energy calculated at the energy calculation step.

**26.** The directed graph layout method according to claim 25, further comprising:

a unit vector multiplication step of multiplying the each unit vector of each of the directed edges by a coefficient according to a type of the each directed edge, wherein
at the synthetic vector generation step, the synthetic vector is generated using the each unit vector multiplied by the coefficient at the unit vector multiplication step.

**27.** The directed graph layout method according to claim 25, wherein
the angle correcting force is a preset constant.

**28.** The directed graph layout method according to claim 25, wherein
the angle correcting force is changed according to the angle.

**29.** The directed graph layout method according to claim 25, further comprising:

an attractive force calculation step of calculating an attractive force generated between the nodes connected to each other by the edges;
a repulsive force calculation step of calculating a repulsive force generated between the nodes within a certain distance, wherein
at the energy calculation step, the energy is calculated by calculating a sum of the attractive forces, the repulsive forces, and the angle correcting forces for all the nodes.

**30.** A program capable of making a computer execute a directed graph layout method for automatically laying out a directed graph configured by nodes and directed edges, comprising:

a synthetic vector generation step of generating a synthetic vector of all unit vectors, the unit vectors being the directed edges connected to each of the nodes;
an angle correcting force generation step of generating an angle correcting force for the each node serving as a connection destination node of the directed edges in a direction in which an angle between the synthetic vector generated at the synthetic vector generation step and each of the unit vectors is smaller and in a direction perpendicular to the each unit vector;
an energy calculation step of calculating an energy by calculating a sum of the angle correcting forces for all the nodes; and
an optimization control step of changing a position of each of the nodes so as to optimize the energy calculated at the energy calculation step.

**31.** The program according to claim 30, further comprising:

a unit vector multiplication step of multiplying the each unit vector of each of the directed edges by a coefficient according to a type of the each directed edge, wherein
at the synthetic vector generation step, the synthetic vector is generated.using the each unit vector multiplied by the coefficient at the unit vector multiplication step.

**32.** The program according to claim 30, wherein
the angle correcting force is a preset constant.

**33.** The directed graph layout program according to claim 30, wherein
the angle correcting force is changed according to the angle.

**34.** The program according to claim 30, further comprising:

an attractive force calculation step of calculating an attractive force generated between the nodes connected to each other by the edges;
a repulsive force calculation step of calculating a repulsive force generated between the nodes within a certain distance, wherein
at the energy calculation step, the energy is calculated by calculating a sum of the attractive forces, the repulsive forces, and the angle correcting forces for all the nodes.

**35.** A computer readable recording medium having recorded therein the program according to any one of claims 30 to 34.

# FIG.1

(BASIC PRINCIPLE OF THE PRESENT INVENTION)

SPRING LAYOUT PROCESSING

DISPLAY OF PATHWAY GRAPH

BINARY RELATION
MODEL DB

NODE

EDGE

ATTRACTIVE
FORCE
CALCULATION

REPULSION
FORCE
CALCULATION

DRAWING
OF GRAPH

REPULSION
FIELD
CALCULATION

BOUNDARY
DETERMINATION

DYNAMIC PARAMETER CONTROL
PROCESSING

EP 1 628 229 A1

# FIG.2

INPUT DEVICE ~112  OUTPUT DEVICE ~114

100

PATHWAY INFORMATION DISPLAY APPARATUS

INPUT AND OUTPUT CONTROL INTERFACE UNIT ~108

STORAGE UNIT ~106

CONTROL UNIT ~102

BINARY RELATION MODEL DATABASE ~106a

GRAPH DATA ~106b

BINARY RELATION MODEL CREATION UNIT ~102a

ATTRACTIVE FORCE CALCULATION UNIT ~102b

REPULSION FORCE CALCULATION UNIT ~102c

REPULSION FIELD CALCULATION UNIT ~102d

BOUNDARY DETERMINATION UNIT ~102e

DYNAMIC PARAMETER CONTROL UNIT ~102f

GRAPH DRAWING UNIT ~102g

ENERGY CALCULATION UNIT ~102h

DUMMY NODE PROCESSING UNIT ~102i

COMMUNICATION CONTROL INTERFACE UNIT ~104

EXTERNAL SYSTEM ~200

EXTERNAL DATABASE

EXTERNAL PROGRAM

NETWORK ~300

CLOCK GENERATOR ~110

EP 1 628 229 A1

# FIG.3

(CONCEPTUAL VIEW OF BINARY RELATION MODEL)

# FIG.4

BINARY RELATION MODEL DATABASE
106a

| CONNECTION SOURCE INFORMATION | CONNECTION DESTINATION INFORMATION | EDGE INFORMATION | RELATION INFORMATION |
|---|---|---|---|
| $A(x_1, y_1)$ | $B(x_2, y_2)$ | $e_1(\ell_1)$ | $\cdots$ |
| $B(x_2, y_2)$ | $C(x_3, y_3)$ | $e_2(\ell_2)$ | $\cdots$ |
| $B(x_2, y_2)$ | $D(x_4, y_4)$ | $e_3(\ell_3)$ | $\cdots$ |

# FIG.5

START

CREATE BINARY RELATION MODEL DB — SA-1

EXECUTE SPRING LAYOUT PROCESSING — SA-2

END

# FIG.6

```
                    ┌─────────────┐
                    │    START    │
                    └──────┬──────┘
                           │
              ┌────────────────────────────┐
              │  ACTIVATE CONTROL TIMER     │──SB-1
              └────────────┬───────────────┘
                           │
                           │        SB-2
                      ◇─────────────◇
              No      │  DOES TIME t1 │     Yes
          ┌───────────│    PASS?     │───────────┐
          │           ◇─────────────◇           │
          │      SB-3                        SB-4│
    ┌─────────────┐                    ┌─────────────┐
    │SET PROXIMITY│                    │SET DISPERSION│
    │PARAMETER TO │                    │PARAMETER TO │
    │DYNAMIC      │                    │DYNAMIC      │
    │PARAMETER    │                    │PARAMETER    │
    └──────┬──────┘                    └──────┬──────┘
           │                                  │
           └────────────┬─────────────────────┘
                        │
              ┌─────────────────────┐
              │CALCULATE REPULSIVE  │
              │FORCE BETWEEN EACH   │──SB-5
              │NODE PAIR            │
              └──────────┬──────────┘
                         │
              ┌─────────────────────┐
              │CALCULATE ATTRCTIVE  │──SB-6
              │FORCE FOR EACH EDGE  │
              └──────────┬──────────┘
                         │
              ┌─────────────────────┐
              │CALCULATE REPULSION  │──SB-7
              │FIELD FOR EACH NODE  │
              └──────────┬──────────┘
                         │
              ┌─────────────────────┐
              │EXECUTE DYNAMIC      │
              │PARAMETER CONTROL    │──SB-8
              │PROCESSING           │
              └──────────┬──────────┘
                         │
              ┌─────────────────────┐
              │OUTPUT DRAWN GRAPH   │──SB-9
              └──────────┬──────────┘
                         │
                         │          SB-10
                    ◇─────────────◇
              No    │    DOES       │
          ┌─────────│USER INDICATE  │
          │         │END OR IS      │
          │         │ENERGY LOWER   │
          │         │THAN THRESHOLD?│
          │         ◇─────────────◇
          │               │Yes
          │         ┌───────────┐
          │         │    END    │
          │         └───────────┘
```

# FIG.7

```
        ┌──────────────┐
        │    START     │
        └──────────────┘
               │
               ▼        SC-1
          ╱───────────╲
         ╱  DETERMINE  ╲
PROXIMITY   DYNAMIC      DISPERSION
         ╲  PARAMETER  ╱
          ╲───────────╱
         │              │
         ▼ SC-2         ▼ SC-3
┌──────────────────┐  ┌──────────────────────┐
│ NARROW REPULSION │  │ WIDEN REPULSION FIELD,│
│ FIELD AND STRENGTHEN│ │ WEAKEN ATTRCTIVE FORCE,│
│ ATTRCTIVE FORCE  │  │ AND WEAKEN REPULSIVE  │
│                  │  │ FORCE                 │
└──────────────────┘  └──────────────────────┘
         │              │
         └──────┬───────┘
                ▼
        ┌──────────────┐
        │     END      │
        └──────────────┘
```

# FIG.8

```
                    START
                      │
                      ▼
┌─────────────────────────────────────────────┐
│   LAY OUT DUMMY NODE AT CENTER OF CONNECTION │
│            DESTINATION EDGES                 │        SD-1
│                                              │
│         A ──── t ────► B                     │
│            e₁                                │
│                 │                            │
│                 e₂                           │
│                 C                            │
└─────────────────────────────────────────────┘
```

LAY OUT DUMMY NODE AT CENTER OF CONNECTION DESTINATION EDGES

$A$ —$e_1$— $t$ ——► $B$

$t$ ↑ $e_2$ $C$

— SD-1

ARRANGE DUMMY EDGES ($e_3$ AND $e_4$) TO BE CONNECTED TO NODES TO WHICH CONNECTION DESTINATION EDGE IS CONNECTED, AND DELETE ORIGINAL EDGE ($e_1$)

$A$ —$e_3$—► $t$ —$e_4$—► $B$

$t$ ↑ $e_2$ $C$

— SD-2

REGARD DUMMY NODE AS NORMAL NODE AND EXECUTE SPRING LAYOUT PROCESSING

— SD-3

DRAW ORIGINAL EDGE SO THAT CENTRAL PORTION OF DUMMY NODE IS A BENT POINT, AND DELETE DUMMY NODE AND DUMMY EDGES

$A$ $e_1$ ► $B$

● ↑ $e_2$ $C$

— SD-4

END

# FIG.9

⟨BINARY RELATION MODEL CREATION SCREEN⟩

PATHWAY NAME [                    ] ⏤ MA-1

| CONNECTION SOURCE INFORMATION (MA-2) | | CONNECTION DESTINATION INFORMATION (MA-3) | | EDGE INFORMATION (MA-4) |
|---|---|---|---|---|
| NAME | INITIAL LAYOUT COORDINATE | NAME | INITIAL LAYOUT COORDINATE | NAME |
|  |  |  |  |  |
|  |  |  |  |  |
|  |  |  |  |  |

# FIG.10

(BASIC PRINCIPLE OF THE PRESENT INVENTION)

DIRECTED GRAPH LAYOUT PROCESSING

DISPLAY OF DIRECTED GRAPH OF PATHWAY INFORMATION

BINARY RELATION MODEL DB

NODE

EDGE

ATTRACTIVE FORCE CALCULATION

REPULSION FORCE CALCULATION

ADJUSTMENT OF ANGLE

ENERGY CALCULATION

DRAWING OF GRAPH

OPTIMIZATION PROCESSING CONTROL

EP 1 628 229 A1

# FIG.11

# FIG.12

# FIG.13

# FIG.14

INPUT DEVICE 1112

OUTPUT DEVICE 1114

1100

**DIRECTED GRAPH LAYOUT UNIT**

INPUT AND OUTPUT CONTROL INTERFACE UNIT 1108

**STORAGE UNIT** 1106

BINARY RELATION MODEL DATABASE 1106a

GRAPH DATA 1106b

**CONTROL UNIT** 1102

BINARY RELATION MODEL CREATION UNIT 1102a

ATTRCTIVE FORCE CALCULATION UNIT 1102b

REPULSION FORCE CALCULATION UNIT 1102c

ANGLE ADJUSTMENT UNIT 1102d

SYNTHETIC VECTOR GENERATION UNIT 1102h

ANGLE CORRECTING FORCE GENERATION UNIT 1102i

ENERGY CALCULATION UNIT 1102e

OPTIMIZATION CONTROL UNIT 1102f

GRAPH DRAWING UNIT 1102g

COMMUNICATION CONTROL INTERFACE UNIT 1104

**EXTERNAL SYSTEM** 1200

EXTERNAL DATABASE

EXTERNAL PROGRAM

NETWORK 1300

EP 1 628 229 A1

# FIG.15

(CONCEPTUAL VIEW OF BINARY RELATION MODEL)

# FIG.16

BINARY RELATION MODEL DATABASE
1106a

| CONNECTION SOURCE INFORMATION | CONNECTION DESTINATION INFORMATION | EDGE INFORMATION | RELATION INFORMATION |
|---|---|---|---|
| $A(x_1, y_1)$ | $B(x_2, y_2)$ | $e_1 (\ell_1)$ | $\cdots$ |
| $B(x_2, y_2)$ | $C(x_3, y_3)$ | $e_2 (\ell_2)$ | $\cdots$ |
| $B(x_2, y_2)$ | $D(x_4, y_4)$ | $e_3 (\ell_3)$ | $\cdots$ |

# FIG.17

```
          ┌──────────┐
          │  START   │
          └────┬─────┘
               │
               ▼
┌─────────────────────────────────────┐
│ CREATE BINARY RELATION MODEL DB     │──SAA-1
└─────────────────────────────────────┘
               │
               ▼
┌─────────────────────────────────────┐
│ EXECUTE DIRECTED GRAPH LAYOUT       │──SAA-2
│            PROCESSING               │
└─────────────────────────────────────┘
               │
               ▼
          ┌──────────┐
          │   END    │
          └──────────┘
```

# FIG.18

```
        ┌─────────────┐
        │    START    │
        └─────────────┘
               │
               ▼
   ┌───────────────────────────┐
   │    MAKE INITIAL LAYOUT     │  ~SBB-1
   └───────────────────────────┘
               │
   ┌──────────▼─────────────────┐
   │  CALCULATE ATTRCTIVE FORCE  │  ~SBB-2
   │  (SPRING ENERGY) FOR EACH NODE │
   └────────────────────────────┘
               │
               ▼
   ┌────────────────────────────┐
   │  CALCULATE REPULSIVE FORCE  │  ~SBB-3
   │ (COULOMB FORCE) FOR EACH NODE │
   └────────────────────────────┘
               │
               ▼
   ┌────────────────────────────┐
   │ CALCULATE ANGLE CORRECTING FORCE │  ~SBB-4
   │        FOR EACH NODE        │
   └────────────────────────────┘
               │
               ▼
   ┌────────────────────────────┐
   │   ADD UP ALL ATTRCTIVE FORCES, │  ~SBB-5
   │  REPULSIVE FORCES, AND ANGLE │
   │ CORRECTING FORCES, AND CALCULATE │
   │    ENERGY OF ENTIRE GRAPH   │
   └────────────────────────────┘
               │
               ▼
   ┌────────────────────────────┐
   │  PERFORM OPTIMIZATION BASED ON │  ~SBB-6
   │  ENERGY AND DETERMINE LAYOUT │
   └────────────────────────────┘
               │
               ▼           SBB-7
          ◇─────────────────◇
     No  │  DOES LAYOUT       │
   ◄─────│  SATISFY END       │
         │  CONDITION?        │
          ◇─────────────────◇
               │ Yes
               ▼
        ┌─────────────┐
        │     END     │
        └─────────────┘
```

# FIG. 19

```
        ┌─────────────┐
        │    START    │
        └──────┬──────┘
               │
               ▼
┌──────────────────────────────┐
│   CREATE SYNTHETIC VECTOR     │──SCC-1
└──────────────┬───────────────┘
               │
               ▼
┌──────────────────────────────┐
│   GENERATE ANGLE CORRECTING   │
│     FORCE FOR CONNECTION      │──SCC-2
│   DESTINATION NODE OF EDGE    │
└──────────────┬───────────────┘
               │
               ▼
        ┌─────────────┐
        │     END     │
        └─────────────┘
```

# FIG.20

# FIG.21

〈BINARY RELATION MODEL CREATION SCREEN〉

PATHWAY NAME [                    ] MAA-1

| CONNECTION SOURCE INFORMATION NAME (MAA-2) | | CONNECTION DESTINATION INFORMATION (MAA-3) | | EDGE INFORMATION (MAA-4) |
|---|---|---|---|---|
| NAME | INITIAL LAYOUT COORDINATE | NAME | INITIAL LAYOUT COORDINATE | NAME |
|  |  |  |  |  |
|  |  |  |  |  |
|  |  |  |  |  |

## INTERNATIONAL SEARCH REPORT

| International application No. |
| --- |
| PCT/JP03/06676 |

| A. CLASSIFICATION OF SUBJECT MATTER |
| --- |
| Int.Cl⁷ G06F17/30, G06T11/20 |

According to International Patent Classification (IPC) or to both national classification and IPC

| B. FIELDS SEARCHED |
| --- |
| Minimum documentation searched (classification system followed by classification symbols)<br>    Int.Cl⁷ G06F17/30, G06T11/20 |

Documentation searched other than minimum documentation to the extent that such documents are included in the fields searched

| | | | |
| --- | --- | --- | --- |
| Jitsuyo Shinan Koho | 1922–1996 | Toroku Jitsuyo Shinan Koho | 1994–2003 |
| Kokai Jitsuyo Shinan Koho | 1971–2003 | Jitsuyo Shinan Toroku Koho | 1996–2003 |

Electronic data base consulted during the international search (name of data base and, where practicable, search terms used)
    JICST FILE(JOIS), WPI, INSPEC(DIALOG)

### C. DOCUMENTS CONSIDERED TO BE RELEVANT

| Category* | Citation of document, with indication, where appropriate, of the relevant passages | Relevant to claim No. |
| --- | --- | --- |
| E,X | JP 2003-167882 A (Seresuta Rekishiko Saienshizu Kabushiki Kaisha),<br>13 June, 2003 (13.06.03),<br>Full text; all drawings<br>(Family: none) | 1-19 |
| E,X | JP 2003-196667 A (Seresuta Rekishiko Saienshizu Kabushiki Kaisha),<br>11 July, 2003 (11.07.03),<br>Full text; all drawings<br>(Family: none) | 20-35 |
| Y | FUKAGAWA et al., "Idenshi Seigyo Network Kochiku Shien no tame no Workbench System no Kaihatsu", The Tissue Culture Engineering, Vol.27, No.2, 25 February, 2001 (25.02.01), pages 68 to 71, particularly, Fig. 3 | 1-19 |

| ☒ Further documents are listed in the continuation of Box C. | ☐ See patent family annex. |
| --- | --- |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    12 September, 2003 (12.09.03) | Date of mailing of the international search report<br>    30 September, 2003 (30.09.03) |
| --- | --- |
| Name and mailing address of the ISA/<br>    Japanese Patent Office | Authorized officer |
| Facsimile No. | Telephone No. |

Form PCT/ISA/210 (second sheet) (July 1998)

**INTERNATIONAL SEARCH REPORT**

| International application No. |
| --- |
| PCT/JP03/06676 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y<br>A | JP 2002-312803 A  (International Business Machines Corp.),<br>25 October, 2002 (25.10.02),<br>Par. Nos. [0003] to [0004]<br>& US 2002/0196292 A1 | 1-19<br>20-35 |

Form PCT/ISA/210 (continuation of second sheet) (July 1998)

# INTERNATIONAL SEARCH REPORT

International application No.

PCT/JP03/06676

**Box I    Observations where certain claims were found unsearchable (Continuation of item 2 of first sheet)**

This international search report has not been established in respect of certain claims under Article 17(2)(a) for the following reasons:

1. ☐ Claims Nos.:

   because they relate to subject matter not required to be searched by this Authority, namely:

2. ☐ Claims Nos.:

   because they relate to parts of the international application that do not comply with the prescribed requirements to such an extent that no meaningful international search can be carried out, specifically:

3. ☐ Claims Nos.:

   because they are dependent claims and are not drafted in accordance with the second and third sentences of Rule 6.4(a).

**Box II    Observations where unity of invention is lacking (Continuation of item 3 of first sheet)**

This International Searching Authority found multiple inventions in this international application, as follows:

      Claims 1-19 relate to a technical feature of deciding node coordinate position by a spring layout according to the repulsion and attraction between nodes and repulsion an attraction of the repulsive field.
      Claims 20-35 relate to a technical feature of generating a composite vector combining all the unit vectors each being a directional edge connected for each node, generating an angle correction force in the direction to minimize the angle between the composite vector generated for the directional edge connection destination node and the unit vector and in the direction vertical to the unit vector, calculating energy by totaling the angle correction forces (Continued to extra sheet)

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐    The additional search fees were accompanied by the applicant's protest.

                         ☒    No protest accompanied the payment of additional search fees.

Form PCT/ISA/210 (continuation of first sheet (1)) (July 1998)

| **INTERNATIONAL SEARCH REPORT** | International application No. |
|---|---|
| | PCT/JP03/06676 |

Continuation of Box No.II of continuation of first sheet(1)

of all the nodes, and modifying the node coordinate position so that the calculated energy is optimal.

No technical relationship within the meaning of PCT Rule 13 between claims 1-19 and claims 20-35 can be seen. Accordingly, the present application includes two groups of inventions: claims 1-19 and claims 20-35.

Form PCT/ISA/210 (extra sheet) (July 1998)